(19) 

Europäisches
Patentamt
European
Patent Office
Office européen
des brevets

(11) **EP 2 471 901 A1**

(12) **EUROPEAN PATENT APPLICATION**
published in accordance with Art. 153(4) EPC

(43) Date of publication:
**04.07.2012 Bulletin 2012/27**

(51) Int Cl.:
*C12N 5/00* (2006.01)  *C12N 5/07* (2010.01)
*C12N 5/10* (2006.01)  *C07K 14/78* (2006.01)

(21) Application number: **10811841.5**

(22) Date of filing: **24.08.2010**

(86) International application number:
**PCT/JP2010/064254**

(87) International publication number:
**WO 2011/024791 (03.03.2011 Gazette 2011/09)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **25.08.2009 JP 2009194444
18.12.2009 JP 2009287258**

(71) Applicant: **Takara Bio, Inc.
Otsu-shi
Shiga 520-2193 (JP)**

(72) Inventors:
• **KATAYAMA, Yoshinori
Otsu-shi
Shiga 520-2193 (JP)**
• **SAKAI, Kensuke
Otsu-shi
Shiga 520-2193 (JP)**

• **YOSHIOKA, Hirofumi
Otsu-shi
Shiga 520-2193 (JP)**
• **TAKAKURA, Hikaru
Otsu-shi
Shiga 520-2193 (JP)**
• **CHONO, Hideto
Otsu-shi
Shiga 520-2193 (JP)**
• **SAITO, Naoki
Otsu-shi
Shiga 520-2193 (JP)**
• **MINENO, Junichi
Otsu-shi
Shiga 520-2193 (JP)**

(74) Representative: **Zwicker, Jörk et al
Dr. Volker Vossius
Patent- und Rechtsanwaltskanzlei
Geibelstrasse 6
81679 München (DE)**

(54) **METHOD FOR PRODUCING T CELL POPULATION UNDER PRESENCE OF RETINOIC ACID**

(57) Disclosed is a method for producing a cell population containing memory-like T cells, said method being **characterized by** including a step for in vitro culturing of a cell population containing T cells or T cell precursor cells using a retinoic acid and a CD3 ligand. Further disclosed is a method for producing a cell population wherein the proportion of memory-like T cells is increased and wherein a desired gene is introduced with a high efficiency and is highly expressed.

**EP 2 471 901 A1**

SEQUENCE LISTING

<110> TAKARA BIO INC.

<120> Process for the preparation of T lymphocytes in the presence of retinoic acid

<130> 669821

<150> JP 2009-194444
<151> 2009-08-25

<150> JP 2009-287258
<151> 2009-12-18

<160> 4

<170> PatentIn version 3.3

<210> 1
<211> 549
<212> PRT
<213> Artificial Sequence

<220>
<223> fibronectin fragment named CH-271

<400> 1

```
Pro Thr Asp Leu Arg Phe Thr Asn Ile Gly Pro Asp Thr Met Arg Val
1               5                   10                  15


Thr Trp Ala Pro Pro Pro Ser Ile Asp Leu Thr Asn Phe Leu Val Arg
            20                  25                  30


Tyr Ser Pro Val Lys Asn Glu Glu Asp Val Ala Glu Leu Ser Ile Ser
        35                  40                  45


Pro Ser Asp Asn Ala Val Val Leu Thr Asn Leu Leu Pro Gly Thr Glu
    50                  55                  60


Tyr Val Val Ser Val Ser Ser Val Tyr Glu Gln His Glu Ser Thr Pro
65                  70                  75                  80


Leu Arg Gly Arg Gln Lys Thr Gly Leu Asp Ser Pro Thr Gly Ile Asp
            85                  90                  95


Phe Ser Asp Ile Thr Ala Asn Ser Phe Thr Val His Trp Ile Ala Pro
            100                 105                 110


Arg Ala Thr Ile Thr Gly Tyr Arg Ile Arg His His Pro Glu His Phe
        115                 120                 125


Ser Gly Arg Pro Arg Glu Asp Arg Val Pro His Ser Arg Asn Ser Ile
    130                 135                 140
```

24

```
Thr Leu Thr Asn Leu Thr Pro Gly Thr Glu Tyr Val Val Ser Ile Val
145                 150                 155                 160


Ala Leu Asn Gly Arg Glu Glu Ser Pro Leu Leu Ile Gly Gln Gln Ser
                165                 170                 175


Thr Val Ser Asp Val Pro Arg Asp Leu Glu Val Val Ala Ala Thr Pro
                180                 185                 190


Thr Ser Leu Leu Ile Ser Trp Asp Ala Pro Ala Val Thr Val Arg Tyr
                195                 200                 205


Tyr Arg Ile Thr Tyr Gly Glu Thr Gly Gly Asn Ser Pro Val Gln Glu
        210                 215                 220


Phe Thr Val Pro Gly Ser Lys Ser Thr Ala Thr Ile Ser Gly Leu Lys
225                 230                 235                 240


Pro Gly Val Asp Tyr Thr Ile Thr Val Tyr Ala Val Thr Gly Arg Gly
                245                 250                 255


Asp Ser Pro Ala Ser Ser Lys Pro Ile Ser Ile Asn Tyr Arg Thr Glu
                260                 265                 270


Ile Asp Lys Pro Ser Met Ala Ile Pro Ala Pro Thr Asp Leu Lys Phe
        275                 280                 285


Thr Gln Val Thr Pro Thr Ser Leu Ser Ala Gln Trp Thr Pro Pro Asn
        290                 295                 300


Val Gln Leu Thr Gly Tyr Arg Val Arg Val Thr Pro Lys Glu Lys Thr
305                 310                 315                 320


Gly Pro Met Lys Glu Ile Asn Leu Ala Pro Asp Ser Ser Ser Val Val
                325                 330                 335


Val Ser Gly Leu Met Val Ala Thr Lys Tyr Glu Val Ser Val Tyr Ala
                340                 345                 350


Leu Lys Asp Thr Leu Thr Ser Arg Pro Ala Gln Gly Val Val Thr Thr
        355                 360                 365


Leu Glu Asn Val Ser Pro Pro Arg Arg Ala Arg Val Thr Asp Ala Thr
        370                 375                 380


Glu Thr Thr Ile Thr Ile Ser Trp Arg Thr Lys Thr Glu Thr Ile Thr
385                 390                 395                 400


Gly Phe Gln Val Asp Ala Val Pro Ala Asn Gly Gln Thr Pro Ile Gln
```

```
                    405                  410                  415


        Arg Thr Ile Lys Pro Asp Val Arg Ser Tyr Thr Ile Thr Gly Leu Gln
                    420              425              430


        Pro Gly Thr Asp Tyr Lys Ile Tyr Leu Tyr Thr Leu Asn Asp Asn Ala
                    435              440              445


        Arg Ser Ser Pro Val Val Ile Asp Ala Ser Thr Ala Ile Asp Ala Pro
            450              455              460


        Ser Asn Leu Arg Phe Leu Ala Thr Thr Pro Asn Ser Leu Leu Val Ser
        465              470              475              480


        Trp Gln Pro Pro Arg Ala Arg Ile Thr Gly Tyr Ile Ile Lys Tyr Glu
                    485              490              495


        Lys Pro Gly Ser Pro Pro Arg Glu Val Val Pro Arg Pro Arg Pro Gly
                    500              505              510


        Val Thr Glu Ala Thr Ile Thr Gly Leu Glu Pro Gly Thr Glu Tyr Thr
                    515              520              525


        Ile Tyr Val Ile Ala Leu Lys Asn Asn Gln Lys Ser Glu Pro Leu Ile
            530              535              540


        Gly Arg Lys Lys Thr
        545


        <210>   2
        <211>   574
        <212>   PRT
        <213>   Artificial Sequence

        <220>
        <223>   fibronectin fragment named CH-296

        <400>   2

        Pro Thr Asp Leu Arg Phe Thr Asn Ile Gly Pro Asp Thr Met Arg Val
        1               5               10              15


        Thr Trp Ala Pro Pro Pro Ser Ile Asp Leu Thr Asn Phe Leu Val Arg
                    20              25              30


        Tyr Ser Pro Val Lys Asn Glu Glu Asp Val Ala Glu Leu Ser Ile Ser
                    35              40              45


        Pro Ser Asp Asn Ala Val Val Leu Thr Asn Leu Leu Pro Gly Thr Glu
            50              55              60
```

Tyr Val Val Ser Val Ser Ser Val Tyr Glu Gln His Glu Ser Thr Pro
65                  70                  75                  80

Leu Arg Gly Arg Gln Lys Thr Gly Leu Asp Ser Pro Thr Gly Ile Asp
                85                  90                  95

Phe Ser Asp Ile Thr Ala Asn Ser Phe Thr Val His Trp Ile Ala Pro
            100                 105                 110

Arg Ala Thr Ile Thr Gly Tyr Arg Ile Arg His His Pro Glu His Phe
        115                 120                 125

Ser Gly Arg Pro Arg Glu Asp Arg Val Pro His Ser Arg Asn Ser Ile
    130                 135                 140

Thr Leu Thr Asn Leu Thr Pro Gly Thr Glu Tyr Val Val Ser Ile Val
145                 150                 155                 160

Ala Leu Asn Gly Arg Glu Glu Ser Pro Leu Leu Ile Gly Gln Gln Ser
                165                 170                 175

Thr Val Ser Asp Val Pro Arg Asp Leu Glu Val Val Ala Ala Thr Pro
            180                 185                 190

Thr Ser Leu Leu Ile Ser Trp Asp Ala Pro Ala Val Thr Val Arg Tyr
        195                 200                 205

Tyr Arg Ile Thr Tyr Gly Glu Thr Gly Gly Asn Ser Pro Val Gln Glu
    210                 215                 220

Phe Thr Val Pro Gly Ser Lys Ser Thr Ala Thr Ile Ser Gly Leu Lys
225                 230                 235                 240

Pro Gly Val Asp Tyr Thr Ile Thr Val Tyr Ala Val Thr Gly Arg Gly
                245                 250                 255

Asp Ser Pro Ala Ser Ser Lys Pro Ile Ser Ile Asn Tyr Arg Thr Glu
            260                 265                 270

Ile Asp Lys Pro Ser Met Ala Ile Pro Ala Pro Thr Asp Leu Lys Phe
        275                 280                 285

Thr Gln Val Thr Pro Thr Ser Leu Ser Ala Gln Trp Thr Pro Pro Asn
    290                 295                 300

Val Gln Leu Thr Gly Tyr Arg Val Arg Val Thr Pro Lys Glu Lys Thr
305                 310                 315                 320

Gly Pro Met Lys Glu Ile Asn Leu Ala Pro Asp Ser Ser Ser Val Val

EP 2 471 901 A1

325 330 335

Val Ser Gly Leu Met Val Ala Thr Lys Tyr Glu Val Ser Val Tyr Ala
340 345 350

Leu Lys Asp Thr Leu Thr Ser Arg Pro Ala Gln Gly Val Val Thr Thr
355 360 365

Leu Glu Asn Val Ser Pro Pro Arg Arg Ala Arg Val Thr Asp Ala Thr
370 375 380

Glu Thr Thr Ile Thr Ile Ser Trp Arg Thr Lys Thr Glu Thr Ile Thr
385 390 395 400

Gly Phe Gln Val Asp Ala Val Pro Ala Asn Gly Gln Thr Pro Ile Gln
405 410 415

Arg Thr Ile Lys Pro Asp Val Arg Ser Tyr Thr Ile Thr Gly Leu Gln
420 425 430

Pro Gly Thr Asp Tyr Lys Ile Tyr Leu Tyr Thr Leu Asn Asp Asn Ala
435 440 445

Arg Ser Ser Pro Val Val Ile Asp Ala Ser Thr Ala Ile Asp Ala Pro
450 455 460

Ser Asn Leu Arg Phe Leu Ala Thr Thr Pro Asn Ser Leu Leu Val Ser
465 470 475 480

Trp Gln Pro Pro Arg Ala Arg Ile Thr Gly Tyr Ile Ile Lys Tyr Glu
485 490 495

Lys Pro Gly Ser Pro Pro Arg Glu Val Val Pro Arg Pro Arg Pro Gly
500 505 510

Val Thr Glu Ala Thr Ile Thr Gly Leu Glu Pro Gly Thr Glu Tyr Thr
515 520 525

Ile Tyr Val Ile Ala Leu Lys Asn Asn Gln Lys Ser Glu Pro Leu Ile
530 535 540

Gly Arg Lys Lys Thr Asp Glu Leu Pro Gln Leu Val Thr Leu Pro His
545 550 555 560

Pro Asn Leu His Gly Pro Glu Ile Leu Asp Val Pro Ser Thr
565 570

<210> 3
<211> 271

28

```
<212>   PRT
<213>   Artificial Sequence

<220>
<223>   fibronectin fragment named H-271

<400>   3

Ala Ile Pro Ala Pro Thr Asp Leu Lys Phe Thr Gln Val Thr Pro Thr
1               5               10              15

Ser Leu Ser Ala Gln Trp Thr Pro Pro Asn Val Gln Leu Thr Gly Tyr
            20              25              30

Arg Val Arg Val Thr Pro Lys Glu Lys Thr Gly Pro Met Lys Glu Ile
        35              40              45

Asn Leu Ala Pro Asp Ser Ser Ser Val Val Val Ser Gly Leu Met Val
    50              55              60

Ala Thr Lys Tyr Glu Val Ser Val Tyr Ala Leu Lys Asp Thr Leu Thr
65              70              75              80

Ser Arg Pro Ala Gln Gly Val Val Thr Thr Leu Glu Asn Val Ser Pro
            85              90              95

Pro Arg Arg Ala Arg Val Thr Asp Ala Thr Glu Thr Thr Ile Thr Ile
        100             105             110

Ser Trp Arg Thr Lys Thr Glu Thr Ile Thr Gly Phe Gln Val Asp Ala
        115             120             125

Val Pro Ala Asn Gly Gln Thr Pro Ile Gln Arg Thr Ile Lys Pro Asp
    130             135             140

Val Arg Ser Tyr Thr Ile Thr Gly Leu Gln Pro Gly Thr Asp Tyr Lys
145             150             155             160

Ile Tyr Leu Tyr Thr Leu Asn Asp Asn Ala Arg Ser Ser Pro Val Val
            165             170             175

Ile Asp Ala Ser Thr Ala Ile Asp Ala Pro Ser Asn Leu Arg Phe Leu
        180             185             190

Ala Thr Thr Pro Asn Ser Leu Leu Val Ser Trp Gln Pro Pro Arg Ala
        195             200             205

Arg Ile Thr Gly Tyr Ile Ile Lys Tyr Glu Lys Pro Gly Ser Pro Pro
        210             215             220

Arg Glu Val Val Pro Arg Pro Arg Pro Gly Val Thr Glu Ala Thr Ile
```

```
                225                    230                    235                    240

        Thr Gly Leu Glu Pro Gly Thr Glu Tyr Thr Ile Tyr Val Ile Ala Leu
                        245                    250                    255

        Lys Asn Asn Gln Lys Ser Glu Pro Leu Ile Gly Arg Lys Lys Thr
                        260                    265                    270

        <210>   4
        <211>   296
        <212>   PRT
        <213>   Artificial Sequence

        <220>
        <223>   fibronectin fragment named H-296

        <400>   4

        Ala Ile Pro Ala Pro Thr Asp Leu Lys Phe Thr Gln Val Thr Pro Thr
        1                   5                   10                  15

        Ser Leu Ser Ala Gln Trp Thr Pro Pro Asn Val Gln Leu Thr Gly Tyr
                        20                  25                  30

        Arg Val Arg Val Thr Pro Lys Glu Lys Thr Gly Pro Met Lys Glu Ile
                        35                  40                  45

        Asn Leu Ala Pro Asp Ser Ser Ser Val Val Val Ser Gly Leu Met Val
                        50                  55                  60

        Ala Thr Lys Tyr Glu Val Ser Val Tyr Ala Leu Lys Asp Thr Leu Thr
        65                  70                  75                  80

        Ser Arg Pro Ala Gln Gly Val Val Thr Thr Leu Glu Asn Val Ser Pro
                        85                  90                  95

        Pro Arg Arg Ala Arg Val Thr Asp Ala Thr Glu Thr Thr Ile Thr Ile
                        100                 105                 110

        Ser Trp Arg Thr Lys Thr Glu Thr Ile Thr Gly Phe Gln Val Asp Ala
                        115                 120                 125

        Val Pro Ala Asn Gly Gln Thr Pro Ile Gln Arg Thr Ile Lys Pro Asp
                        130                 135                 140

        Val Arg Ser Tyr Thr Ile Thr Gly Leu Gln Pro Gly Thr Asp Tyr Lys
        145                 150                 155                 160

        Ile Tyr Leu Tyr Thr Leu Asn Asp Asn Ala Arg Ser Ser Pro Val Val
                        165                 170                 175
```

```
Ile Asp Ala Ser Thr Ala Ile Asp Ala Pro Ser Asn Leu Arg Phe Leu
        180             185             190

Ala Thr Thr Pro Asn Ser Leu Leu Val Ser Trp Gln Pro Pro Arg Ala
        195             200             205

Arg Ile Thr Gly Tyr Ile Ile Lys Tyr Glu Lys Pro Gly Ser Pro Pro
    210             215             220

Arg Glu Val Val Pro Arg Pro Arg Pro Gly Val Thr Glu Ala Thr Ile
225             230             235             240

Thr Gly Leu Glu Pro Gly Thr Glu Tyr Thr Ile Tyr Val Ile Ala Leu
            245             250             255

Lys Asn Asn Gln Lys Ser Glu Pro Leu Ile Gly Arg Lys Lys Thr Asp
        260             265             270

Glu Leu Pro Gln Leu Val Thr Leu Pro His Pro Asn Leu His Gly Pro
        275             280             285

Glu Ile Leu Asp Val Pro Ser Thr
        290             295
```

SEQUENCE LISTING

<110> TAKARA BIO INC.

<120> Process for the preparation of T lymphocytes in the presence of retinoic acid

<130> 38-137

<150> JP 2009-194444
<151> 2009-08-25

<150> JP 2009-287258
<151> 2009-12-18

<160> 4

<170> PatentIn version 3.3

<210> 1
<211> 549
<212> PRT
<213> Artificial Sequence

<220>
<223> fibronectin fragment named CH-271

<400> 1

Pro Thr Asp Leu Arg Phe Thr Asn Ile Gly Pro Asp Thr Met Arg Val
1               5                   10                  15

Thr Trp Ala Pro Pro Pro Ser Ile Asp Leu Thr Asn Phe Leu Val Arg
            20                  25                  30

Tyr Ser Pro Val Lys Asn Glu Glu Asp Val Ala Glu Leu Ser Ile Ser
        35                  40                  45

Pro Ser Asp Asn Ala Val Val Leu Thr Asn Leu Leu Pro Gly Thr Glu
    50                  55                  60

Tyr Val Val Ser Val Ser Ser Val Tyr Glu Gln His Glu Ser Thr Pro
65                  70                  75                  80

Leu Arg Gly Arg Gln Lys Thr Gly Leu Asp Ser Pro Thr Gly Ile Asp
            85                  90                  95

Phe Ser Asp Ile Thr Ala Asn Ser Phe Thr Val His Trp Ile Ala Pro
            100                 105                 110

Arg Ala Thr Ile Thr Gly Tyr Arg Ile Arg His His Pro Glu His Phe
            115                 120                 125

Ser Gly Arg Pro Arg Glu Asp Arg Val Pro His Ser Arg Asn Ser Ile
        130                 135                 140

```
Thr Leu Thr Asn Leu Thr Pro Gly Thr Glu Tyr Val Val Ser Ile Val
145                 150                 155                 160


Ala Leu Asn Gly Arg Glu Glu Ser Pro Leu Leu Ile Gly Gln Gln Ser
                165                 170                 175


Thr Val Ser Asp Val Pro Arg Asp Leu Glu Val Val Ala Ala Thr Pro
                180                 185                 190


Thr Ser Leu Leu Ile Ser Trp Asp Ala Pro Ala Val Thr Val Arg Tyr
                195                 200                 205


Tyr Arg Ile Thr Tyr Gly Glu Thr Gly Gly Asn Ser Pro Val Gln Glu
                210                 215                 220


Phe Thr Val Pro Gly Ser Lys Ser Thr Ala Thr Ile Ser Gly Leu Lys
225                 230                 235                 240


Pro Gly Val Asp Tyr Thr Ile Thr Val Tyr Ala Val Thr Gly Arg Gly
                245                 250                 255


Asp Ser Pro Ala Ser Ser Lys Pro Ile Ser Ile Asn Tyr Arg Thr Glu
                260                 265                 270


Ile Asp Lys Pro Ser Met Ala Ile Pro Ala Pro Thr Asp Leu Lys Phe
                275                 280                 285


Thr Gln Val Thr Pro Thr Ser Leu Ser Ala Gln Trp Thr Pro Pro Asn
                290                 295                 300


Val Gln Leu Thr Gly Tyr Arg Val Arg Val Thr Pro Lys Glu Lys Thr
305                 310                 315                 320


Gly Pro Met Lys Glu Ile Asn Leu Ala Pro Asp Ser Ser Ser Val Val
                325                 330                 335


Val Ser Gly Leu Met Val Ala Thr Lys Tyr Glu Val Ser Val Tyr Ala
                340                 345                 350


Leu Lys Asp Thr Leu Thr Ser Arg Pro Ala Gln Gly Val Val Thr Thr
                355                 360                 365


Leu Glu Asn Val Ser Pro Pro Arg Arg Ala Arg Val Thr Asp Ala Thr
                370                 375                 380


Glu Thr Thr Ile Thr Ile Ser Trp Arg Thr Lys Thr Glu Thr Ile Thr
385                 390                 395                 400


Gly Phe Gln Val Asp Ala Val Pro Ala Asn Gly Gln Thr Pro Ile Gln
```

```
                        405                     410                     415


      Arg Thr Ile Lys Pro Asp Val Arg Ser Tyr Thr Ile Thr Gly Leu Gln
                  420                 425             430


      Pro Gly Thr Asp Tyr Lys Ile Tyr Leu Tyr Thr Leu Asn Asp Asn Ala
                  435                 440             445


      Arg Ser Ser Pro Val Val Ile Asp Ala Ser Thr Ala Ile Asp Ala Pro
          450                 455             460


      Ser Asn Leu Arg Phe Leu Ala Thr Thr Pro Asn Ser Leu Leu Val Ser
      465                 470                 475                 480


      Trp Gln Pro Pro Arg Ala Arg Ile Thr Gly Tyr Ile Ile Lys Tyr Glu
                  485                 490             495


      Lys Pro Gly Ser Pro Pro Arg Glu Val Val Pro Arg Pro Arg Pro Gly
                  500                 505             510


      Val Thr Glu Ala Thr Ile Thr Gly Leu Glu Pro Gly Thr Glu Tyr Thr
                  515                 520             525


      Ile Tyr Val Ile Ala Leu Lys Asn Asn Gln Lys Ser Glu Pro Leu Ile
                  530                 535             540


      Gly Arg Lys Lys Thr
      545


      <210>   2
      <211>   574
      <212>   PRT
      <213>   Artificial Sequence

      <220>
      <223>   fibronectin fragment named CH-296

      <400>   2

      Pro Thr Asp Leu Arg Phe Thr Asn Ile Gly Pro Asp Thr Met Arg Val
      1               5                   10              15


      Thr Trp Ala Pro Pro Pro Ser Ile Asp Leu Thr Asn Phe Leu Val Arg
                  20                  25                  30


      Tyr Ser Pro Val Lys Asn Glu Glu Asp Val Ala Glu Leu Ser Ile Ser
                  35                  40                  45


      Pro Ser Asp Asn Ala Val Val Leu Thr Asn Leu Leu Pro Gly Thr Glu
          50                  55                  60
```

```
Tyr Val Val Ser Val Ser Ser Val Tyr Glu Gln His Glu Ser Thr Pro
65              70              75                      80


Leu Arg Gly Arg Gln Lys Thr Gly Leu Asp Ser Pro Thr Gly Ile Asp
            85              90                  95


Phe Ser Asp Ile Thr Ala Asn Ser Phe Thr Val His Trp Ile Ala Pro
            100             105             110


Arg Ala Thr Ile Thr Gly Tyr Arg Ile Arg His His Pro Glu His Phe
            115             120             125


Ser Gly Arg Pro Arg Glu Asp Arg Val Pro His Ser Arg Asn Ser Ile
    130             135             140


Thr Leu Thr Asn Leu Thr Pro Gly Thr Glu Tyr Val Val Ser Ile Val
145             150             155             160


Ala Leu Asn Gly Arg Glu Glu Ser Pro Leu Leu Ile Gly Gln Gln Ser
            165             170             175


Thr Val Ser Asp Val Pro Arg Asp Leu Glu Val Val Ala Ala Thr Pro
            180             185             190


Thr Ser Leu Leu Ile Ser Trp Asp Ala Pro Ala Val Thr Val Arg Tyr
            195             200             205


Tyr Arg Ile Thr Tyr Gly Glu Thr Gly Gly Asn Ser Pro Val Gln Glu
    210             215             220


Phe Thr Val Pro Gly Ser Lys Ser Thr Ala Thr Ile Ser Gly Leu Lys
225             230             235             240


Pro Gly Val Asp Tyr Thr Ile Thr Val Tyr Ala Val Thr Gly Arg Gly
            245             250             255


Asp Ser Pro Ala Ser Ser Lys Pro Ile Ser Ile Asn Tyr Arg Thr Glu
            260             265             270


Ile Asp Lys Pro Ser Met Ala Ile Pro Ala Pro Thr Asp Leu Lys Phe
    275             280             285


Thr Gln Val Thr Pro Thr Ser Leu Ser Ala Gln Trp Thr Pro Pro Asn
    290             295             300


Val Gln Leu Thr Gly Tyr Arg Val Arg Val Thr Pro Lys Glu Lys Thr
305             310             315             320


Gly Pro Met Lys Glu Ile Asn Leu Ala Pro Asp Ser Ser Ser Val Val
```

```
                    325                    330                         335

        Val Ser Gly Leu Met Val Ala Thr Lys Tyr Glu Val Ser Val Tyr Ala
                340                345                350

        Leu Lys Asp Thr Leu Thr Ser Arg Pro Ala Gln Gly Val Val Thr Thr
                355                360                365

        Leu Glu Asn Val Ser Pro Pro Arg Arg Ala Arg Val Thr Asp Ala Thr
            370                375                380

        Glu Thr Thr Ile Thr Ile Ser Trp Arg Thr Lys Thr Glu Thr Ile Thr
        385                390                395                400

        Gly Phe Gln Val Asp Ala Val Pro Ala Asn Gly Gln Thr Pro Ile Gln
                    405                410                415

        Arg Thr Ile Lys Pro Asp Val Arg Ser Tyr Thr Ile Thr Gly Leu Gln
                420                425                430

        Pro Gly Thr Asp Tyr Lys Ile Tyr Leu Tyr Thr Leu Asn Asp Asn Ala
                435                440                445

        Arg Ser Ser Pro Val Val Ile Asp Ala Ser Thr Ala Ile Asp Ala Pro
            450                455                460

        Ser Asn Leu Arg Phe Leu Ala Thr Thr Pro Asn Ser Leu Leu Val Ser
        465                470                475                480

        Trp Gln Pro Pro Arg Ala Arg Ile Thr Gly Tyr Ile Ile Lys Tyr Glu
                    485                490                495

        Lys Pro Gly Ser Pro Pro Arg Glu Val Val Pro Arg Pro Arg Pro Gly
                500                505                510

        Val Thr Glu Ala Thr Ile Thr Gly Leu Glu Pro Gly Thr Glu Tyr Thr
                515                520                525

        Ile Tyr Val Ile Ala Leu Lys Asn Asn Gln Lys Ser Glu Pro Leu Ile
                530                535                540

        Gly Arg Lys Lys Thr Asp Glu Leu Pro Gln Leu Val Thr Leu Pro His
        545                550                555                560

        Pro Asn Leu His Gly Pro Glu Ile Leu Asp Val Pro Ser Thr
                    565                570


        <210>  3
        <211>  271
```

<212> PRT
<213> Artificial Sequence

<220>
<223> fibronectin fragment named H-271

<400> 3

```
Ala Ile Pro Ala Pro Thr Asp Leu Lys Phe Thr Gln Val Thr Pro Thr
1               5                   10                  15

Ser Leu Ser Ala Gln Trp Thr Pro Pro Asn Val Gln Leu Thr Gly Tyr
            20                  25                  30

Arg Val Arg Val Thr Pro Lys Glu Lys Thr Gly Pro Met Lys Glu Ile
            35                  40                  45

Asn Leu Ala Pro Asp Ser Ser Ser Val Val Val Ser Gly Leu Met Val
        50                  55                  60

Ala Thr Lys Tyr Glu Val Ser Val Tyr Ala Leu Lys Asp Thr Leu Thr
65                  70                  75                  80

Ser Arg Pro Ala Gln Gly Val Val Thr Thr Leu Glu Asn Val Ser Pro
            85                  90                  95

Pro Arg Arg Ala Arg Val Thr Asp Ala Thr Glu Thr Thr Ile Thr Ile
            100                 105                 110

Ser Trp Arg Thr Lys Thr Glu Thr Ile Thr Gly Phe Gln Val Asp Ala
            115                 120                 125

Val Pro Ala Asn Gly Gln Thr Pro Ile Gln Arg Thr Ile Lys Pro Asp
    130                 135                 140

Val Arg Ser Tyr Thr Ile Thr Gly Leu Gln Pro Gly Thr Asp Tyr Lys
145                 150                 155                 160

Ile Tyr Leu Tyr Thr Leu Asn Asp Asn Ala Arg Ser Ser Pro Val Val
                165                 170                 175

Ile Asp Ala Ser Thr Ala Ile Asp Ala Pro Ser Asn Leu Arg Phe Leu
            180                 185                 190

Ala Thr Thr Pro Asn Ser Leu Leu Val Ser Trp Gln Pro Pro Arg Ala
            195                 200                 205

Arg Ile Thr Gly Tyr Ile Ile Lys Tyr Glu Lys Pro Gly Ser Pro Pro
        210                 215                 220

Arg Glu Val Val Pro Arg Pro Arg Pro Gly Val Thr Glu Ala Thr Ile
```

37

```
            225                 230                 235                 240

Thr Gly Leu Glu Pro Gly Thr Glu Tyr Thr Ile Tyr Val Ile Ala Leu
                245                 250                 255

Lys Asn Asn Gln Lys Ser Glu Pro Leu Ile Gly Arg Lys Lys Thr
                260                 265                 270


<210>   4
<211>   296
<212>   PRT
<213>   Artificial Sequence

<220>
<223>   fibronectin fragment named H-296

<400>   4

Ala Ile Pro Ala Pro Thr Asp Leu Lys Phe Thr Gln Val Thr Pro Thr
1               5                   10                  15


Ser Leu Ser Ala Gln Trp Thr Pro Pro Asn Val Gln Leu Thr Gly Tyr
                20                  25                  30


Arg Val Arg Val Thr Pro Lys Glu Lys Thr Gly Pro Met Lys Glu Ile
            35                  40                  45


Asn Leu Ala Pro Asp Ser Ser Ser Val Val Val Ser Gly Leu Met Val
        50                  55                  60


Ala Thr Lys Tyr Glu Val Ser Val Tyr Ala Leu Lys Asp Thr Leu Thr
65                  70                  75                  80


Ser Arg Pro Ala Gln Gly Val Val Thr Thr Leu Glu Asn Val Ser Pro
                85                  90                  95


Pro Arg Arg Ala Arg Val Thr Asp Ala Thr Glu Thr Thr Ile Thr Ile
                100                 105                 110


Ser Trp Arg Thr Lys Thr Glu Thr Ile Thr Gly Phe Gln Val Asp Ala
            115                 120                 125


Val Pro Ala Asn Gly Gln Thr Pro Ile Gln Arg Thr Ile Lys Pro Asp
        130                 135                 140


Val Arg Ser Tyr Thr Ile Thr Gly Leu Gln Pro Gly Thr Asp Tyr Lys
145                 150                 155                 160


Ile Tyr Leu Tyr Thr Leu Asn Asp Asn Ala Arg Ser Ser Pro Val Val
                165                 170                 175
```

```
Ile Asp Ala Ser Thr Ala Ile Asp Ala Pro Ser Asn Leu Arg Phe Leu
        180                 185                 190

Ala Thr Thr Pro Asn Ser Leu Leu Val Ser Trp Gln Pro Pro Arg Ala
        195                 200                 205

Arg Ile Thr Gly Tyr Ile Ile Lys Tyr Glu Lys Pro Gly Ser Pro Pro
        210                 215                 220

Arg Glu Val Val Pro Arg Pro Arg Pro Gly Val Thr Glu Ala Thr Ile
225                 230                 235                 240

Thr Gly Leu Glu Pro Gly Thr Glu Tyr Thr Ile Tyr Val Ile Ala Leu
                245                 250                 255

Lys Asn Asn Gln Lys Ser Glu Pro Leu Ile Gly Arg Lys Lys Thr Asp
        260                 265                 270

Glu Leu Pro Gln Leu Val Thr Leu Pro His Pro Asn Leu His Gly Pro
        275                 280                 285

Glu Ile Leu Asp Val Pro Ser Thr
        290                 295
```

**Description**

Technical Field

The present invention relates to a process for preparing a cell population containing a memory T-like cell, which is useful in the medical field, particularly, immunotherapies. The present invention also relates to a process for preparing a cell population containing a T cell into which a desired gene is transferred with a high efficiency, which is useful in the medical field.

The present application claims priority to Japanese Patent Application No. 2009-194444 filed on August 25, 2009 and Japanese Patent Application No. 2009-287258 filed on December 18, 2009, both of which are incorporated herein in their entireties.

Background Art

A living body is protected from foreign substances mainly by immunological responses, and the immune system is made up of various cells and soluble factors produced by them. Among the various cells, a leukocyte, particularly a lymphocyte plays a central role. Lymphocytes are classified into two main types called B lymphocytes (hereinafter, referred to as B cells in some cases) and T lymphocytes (hereinafter, referred to as T cells in some cases), and both of them specifically recognize an antigen and act on the antigen to defend a living body.

In the periphery, the majority of T cells are occupied by CD (Cluster of Differentiation) 4 positive T cells having a CD4 marker and CD8 positive T cells having a CD8 marker. The majority of the CD4 positive T cells, which are called helper T (hereinafter, referred to as Th) cells, are involved in assistance of antibody production and induction of a variety of immunological responses, and differentiate into a Th1 type, a Th2 type, and the like depending on different kinds of cytokines produced from them by antigen stimulation. The majority of the CD8 positive T cells, which are called cytotoxic T cells [Tc: cytotoxic T lymphocytes, also called killer T cells, hereinafter referred to as CTLs in some cases], exhibit cytotoxic activity by antigen stimulation and differentiate into a Tc1 type producing type I cytokines such as interferon-y (IFN-γ) and interleukin-2 (IL-2) or a Tc2 type producing type II cytokines such as IL-4 and IL-10.

Some CTLs can recognize a complex in which a major histocompatibility complex (hereinafter, abbreviated as a MHC) class I molecule (in the case of a human, called a human leukocyte antigen class I molecule, hereinafter abbreviated as a HLA class I molecule) encoded in a MHC and an antigen are bound together, via a specific T cell receptor (hereinafter, abbreviated as a TCR), and damage cells presenting the complex on the cell surfaces.

As a fourth cancer therapy next to surgical operations, chemotherapies and radiation therapies, immunotherapies have been recently attracting attention. Since immunotherapies utilize the immunological capacity originally possessed by a human, it is said that physical strain on patients is light as compared with other therapies. Known immunotherapies include a therapy which comprises transfer of cells such as lymphokine activated cells, NKT cells or γδT cells which are obtained from CTLs induced ex vivo or peripheral blood lymphocytes by expansion culture using a variety of methods; a dendritic cell-transferring therapy and a peptide vaccine therapy by which induction of an antigen-specific CTL in vivo is expected; a Th1 cell therapy; and an immunological gene therapy which comprises transferring ex vivo a gene by which a variety of effects can be expected, into these cells, and transferring the cells into a body.

The lymphokine activated cell (LAK cell) is a functional cell population having cytotoxic activity, obtained by adding IL-2 to peripheral blood (peripheral blood leukocytes), umbilical blood, tissue fluid and the like containing lymphocytes, followed by in vitro culture for several days. The proliferation of a LAK cell is further accelerated by adding an anti-CD3 antibody to the culture. The LAK cell thus obtained nonspecifically has toxic activity on a variety of cancer cells and other targets.

For the immunotherapy comprising transfer of the LAK cell, there are problems such as how cytotoxic activity is maintained during expansion culture of an ex vivo induced antigen-specific CTL, and how efficiently a lymphocyte can be expansion-cultured ex vivo. For solving such problems, the effect of use of fibronectin or a fragment thereof has been studied (e.g. Patent Literatures 1 to 3).

A memory T cell is distinguished from a naive T cell by analysis of its cell surface antigen. A memory T cell can be also distinguished from a naive T cell by difference in their functions. That is, the memory T cell can more rapidly differentiate into an effector cell by weaker antigen stimulation, as compared with the naive T cell (Non-Patent Literature 1). Further, the memory T cell is functionally different from the naive T cell, in that one effector cell differentiated from the memory T cell can produce a plurality of cytokines simultaneously. However, the molecular mechanism of memory T cell differentiation is unknown.

The memory T cell includes two different cell populations called a central memory T cell and an effector memory T cell, based on difference in the homing ability or the effector function (Non-Patent Literature 2).

In recent years, it has been reported that, in an immunotherapy, a far higher therapeutic effect can be expected by administration, to a living body, of the naive T cell or the central memory T cell which is in a more undifferentiated state, than the effector T cell which is already terminally differentiated (e.g. Non-Patent Literatures 3 and 4).

In the case of the immunological gene therapy, it can be expected that cytotoxic activity specific for a target antigen is imparted, for example, by transferring a TCR gene capable of recognizing the target antigen into a T cell including a CTL. Based on this expectation, a gene therapy with a TCR gene targeting a variety of antigens such as MART1, gp100 and mHAG HA-2 antigens has been attempted. Further, a gene therapy with a gene encoding a chimeric receptor obtained by combining a human-derived TCR, and a TCR derived from an organism other than a human, as well as an antigen recognition site of an antibody recognizing a target antigen, a group of molecules which associate with a TCR to form an antigen recognition complex (e.g. CD3), a T cell surface antigen (e.g. CD8, CD28) and a part of them, as a receptor to be transferred into a T cell, has been attempted.

A variety of vectors are used in gene transfer. Many of gene therapies which has been studied for clinical application to a human use recombinant retroviral vectors (e.g. replication ability-defective retroviral vectors). Since the retroviral vector efficiently transfer a desired foreign gene into a cell and stably incorporates the gene into the chromosomal DNA, the retroviral vector is a preferable gene transfer means for particularly a gene therapy by which gene expression over a long term is desired.

Fibronectin is a huge glycoprotein having a molecular weight of 250,000 present in blood, on a cell surface, and in an extracellular matrix of a tissue, of an animal, and is known to have various functions. A gene transfer method using a functional substance such as fibronectin or a fragment thereof allows gene transfer with a high efficiency, without co-culturing a retrovirus producer cell and a target cell (e.g. Patent Literature 4). It is believed that improvement in a gene transfer efficiency by the above method results from the fact that the presence of the functional substance allows the retrovirus and the target cell to be placed in the state where they are neighbored, thereby an opportunity for them to interact is increased.

In such gene transfer using a retrovirus, infection of a target cell with the retrovirus results in transfer of a gene. However, a gene transfer efficiency with a retrovirus is not satisfactory yet from a viewpoint of actual clinical application, and further improvement in an infection efficiency is strongly desired.

It has been already reported that a retinoic acid, a vitamin A metabolite, is a physiological factor which imprints homing specificity to a small intestine tissue on a T cell (Patent Literature 5). However, there is no teaching or suggestion that a gene transfer efficiency into a cultured cell is improved by culturing a T cell in the presence of a retinoic acid.

Prior Art References

Patent Literatures

[Patent Literature 1] WO03/016511
[Patent Literature 2] WO03/080817
[Patent Literature 3] WO2005/019450
[Patent Literature 4] WO00/018
[Patent Literature 5] JP-A 2005-336062

Non-Patent Literatures

[Non-Patent Literature 1] Nat. Immunol., vol. 1, pp. 47-53, 2000
[Non-Patent Literature 2] Nature, vol. 401, pp. 708-712, 1999
[Non-Patent Literature 3] J. Clin. Invest., vol. 115, No. 6, pp. 1616-1626, 2005
[Non-Patent Literature 4] J. Immunol., vol. 175, No. 2, pp. 739-748, 2005

Disclosure of Invention

Problems to be Solved by the Invention

An object of the present invention is to provide a process for preparing a cell population containing a high proportion of a memory T-like cell which comprises culture in the presence of a retinoic acid, which is suitable for medical use such as a cancer immunotherapy, and the cell population obtained by the process, which is effective in administration to a living body.

Means for Solving the Problems

The present inventors intensively made efforts in order to solve the above-mentioned problems. As a result, they found that a cell population not only in which a desired gene is transferred with a high efficiency and is highly expressed, but also which has a high content of a memory T-like cell can be obtained by ex vivo culturing a cell population containing a T cell or a precursor cell of a T cell using a retinoic acid and a CD3 ligand. Thus the present invention was completed.

An outline of the present invention is given below. A first aspect of the present invention relates to a process for preparing a cell population containing a memory T-like cell, comprising a step of ex vivo culturing a cell population containing a T cell or a precursor cell of a T cell using a retinoic acid and a CD3 ligand.

The process for preparing a cell population containing a memory T-like cell of the present invention also includes a process according to the first aspect of the present invention wherein the step of ex vivo culturing a cell population containing a T cell or a precursor cell of a T cell is any step selected from (1) to (3):

(1) a step of culturing a cell population containing a T cell or a precursor cell of a T cell in the presence of a retinoic acid and a CD3 ligand,

(2) a step of culturing a cell population containing a T cell or a precursor cell of a T cell in the presence of a retinoic acid and a CD3 ligand and, then, culturing the cell population in the absence of a retinoic acid and a CD3 ligand, and

(3) a step of culturing a cell population containing a T cell or a precursor cell of a T cell in the presence of a CD3 ligand and in the absence of a retinoic acid and, then, culturing the cell population in the presence of a retinoic acid.

In the first aspect of the present invention, the step of ex vivo culturing a cell population containing a T cell or a precursor cell of a T cell may be carried out in the presence of fibronectin or a fragment thereof or a mixture thereof. In addition, the cell population containing a T cell or a precursor cell of a T cell may be a peripheral blood mononuclear cell. The process for preparing a cell population containing a memory T-like cell of the present invention also includes a process further comprising a step of selectively recovering a memory T-like cell from the resulting cell population. According to the first aspect of the present invention, there is provided a cell population for cell therapy, which can rapidly differentiate into a cell having cytotoxic activity in response to even weak antigen stimulation.

A second aspect of the present invention relates to a cell population prepared by the first aspect of the present invention.

A third aspect of the present invention relates to a process for preparing a cell population into which a desired gene is transferred, comprising the steps of:

(a) a step of culturing a cell population containing a T cell or a precursor cell of a T cell by the process according to claim 1, and

(b) a step of transferring the desired gene into the cell population during the step (a) or after completion of the step (a).

The third aspect of the present invention includes a preparation process which is carried out by any one of the following steps (1) to (3):

(1) a step of culturing a cell population containing a T cell or a precursor cell of a T cell in the presence of a retinoic acid and a CD3 ligand, and transferring a desired gene into the resulting cell,

(2) a step of culturing a cell population containing a T cell or a precursor cell of a T cell in the presence of a retinoic acid and a CD3 ligand, transferring a desired gene into the resulting cell population, and then culturing the cell population in the absence of a retinoic acid and a CD3 ligand, and

(3) a step of culturing a cell population containing a T cell or a precursor cell of a T cell in the presence of a CD3 ligand and in the absence of a retinoic acid, transferring a desired gene into the resulting cell population, and then culturing the cell population in the presence of a retinoic acid.

In the third aspect of the present invention, the step of ex vivo culturing a cell population containing a T cell or a precursor cell of a T cell may be carried out in the presence of fibronectin or a fragment thereof or a mixture thereof. The process for preparing a cell population into which a desired gene is transferred of the present invention also includes a process wherein the desired gene is transferred by a retrovirus vector, and a process wherein the desired gene is transferred in the presence of fibronectin or a fragment thereof or a mixture thereof. In addition, the desired gene may be a gene encoding a receptor recognizing an antigen. The receptor recognizing an antigen may be a T cell receptor. The process for preparing a cell population into which a desired gene is transferred of the present invention also includes a process wherein the cell population containing a T cell or a precursor cell of a T cell is a peripheral blood mononuclear cell. According to the third aspect of the present invention, there is provided a cell population for cell therapy, which has an increased proportion of a memory T-like cell and in which a desired gene is transferred with a high efficiency and is highly expressed.

A fourth aspect of the present invention relates to a T cell population into which a desired gene is transferred, obtained by the third aspect of the present invention.

Effect of the Invention

According to the present invention, there are provided a process for preparing a cell population containing a memory T-like cell, which is suitable for use in an immunotherapy, and a process for preparing a cell population which has an increased proportion of a memory T-like cell, and in which a desired gene is transferred with a high efficiency and is highly expressed. The cell population obtained by the process is extremely useful in treating a disease by cell therapy.

Brief Description of Drawings

[Fig. 1] Fig. 1 is a graph showing distribution of a cell surface marker on the 4th day.
[Fig. 2] Fig. 2 is a graph showing a cell growth rate.
[Fig. 3] Fig. 3 is a graph showing distribution of a cell surface marker on the 4th day.
[Fig. 4] Fig. 4 is a graph showing distribution of a cell surface marker on the 7th day.
[Fig. 5] Fig. 5 is a graph showing an expression rate of a transferred gene in CD8 positive cells.
[Fig. 6] Fig. 6 is a graph showing the copy number of a transferred gene in gene-transferred cells.
[Fig. 7] Fig. 7 is a graph showing a cell growth rate.
[Fig. 8] Fig. 8 is a graph showing distribution of a cell surface marker on the 7th day.
[Fig. 9] Fig. 9 is a graph showing an expression rate of a transferred gene on the 7th day by a transfer method using a virus-bound plate for gene transfer.
[Fig. 10] Fig. 10 is a graph showing an expression rate of a transferred gene on the 6th day by a transfer method using polybrene.
[Fig. 11] Fig. 11 is a graph showing the copy number of a transferred gene in gene-transferred cells.
[Fig. 12] Fig. 12 is a graph showing an expression rate of a transferred gene in CD8 single positive cells.
[Fig. 13] Fig. 13 is a graph showing results of measurement of a proportion of IL-4-producing and IFN-$\gamma$-nonproducing cells in CD8 single positive cells.
[Fig. 14] Fig. 14 is a graph showing results of measurement of a proportion of perforin-producing cells in CD8 single positive cells.
[Fig. 15] Fig. 15 is a graph showing specific cytotoxic activity on a MAGE-A4-derived peptide p143 at each E/T ratio in gene-transferred cells.
[Fig. 16] Fig. 16 is a graph showing a proportion of tetramer positive cells, that is, an expression rate of a transferred gene in CD8 single positive cells.
[Fig. 17] Fig. 17 is a graph showing distribution of a cell surface marker in CD8 single positive cells on the 11th day.
[Fig. 18] Fig. 18 is a graph showing distribution of a cell surface marker in tetramer positive and CD8 single positive cells on the 11th day.
[Fig. 19] Fig. 19 is a graph showing results of measurement of a proportion of IL-4-producing or -nonproducing cells in CD8 single positive cells, and a proportion of IFN-$\gamma$-producing or -nonproducing cells in CD8 single positive cells.
[Fig. 20] Fig. 20 is a graph showing a ratio between a proportion of IL-4-producing and IFN-$\gamma$-nonproducing cells in CD8 single positive cells (Tc2) and a proportion of IL-4-nonproducing and IFN-$\gamma$-producing cells in CD8 single positive cells (Tc1).
[Fig. 21] Fig. 21 is a graph showing results of measurement of a proportion of perforin-producing cells in CD8 single positive cells.
[Fig. 22] Fig. 22 is a graph showing specific cytotoxic activity on a MAGE-A4-derived peptide p143 in gene-transferred cells.
[Fig. 23] Fig. 23 is a graph showing a proportion of tetramer positive cells, that is, an expression rate of a transferred gene in CD8 single positive cells.
[Fig. 24] Fig. 24 is a graph showing the expression amount of a tetramer in tetramer positive and CD8 single positive cells, expressed as a value of a mean fluorescence intensity.
[Fig. 25] Fig. 25 is a graph showing the copy number of a transferred gene in gene-transferred cells.
[Fig. 26] Fig. 26 is a graph showing distribution of a cell surface marker in CD4 single positive cells on the 10th day.
[Fig. 27] Fig. 27 is a graph showing results of measurement of a proportion of IL-4-producing and IFN-$\gamma$-nonproducing

cells, a proportion of IL-4-producing and IFN-γ-producing cells, and a proportion of IL-4-nonproducing and IFN-γ-producing cells, in CD4 single positive cells.

[Fig. 28] Fig. 28 is a graph showing results of measurement of a proportion of IL-4-producing cells and a proportion of IFN-γ-producing cells, in CD4 single positive cells.

[Fig. 29] Fig. 29 is a graph showing a ratio between a proportion of IL-4-producing and IFN-γ-nonproducing cells in CD8 single positive cells (Th2) and a proportion of IL-4-nonproducing and IFN-γ-producing cells in CD4 single positive cells (Th1).

Mode for Carrying Out the Invention

In the present invention, a "retinoic acid" is also called vitamin A acid, and may be either all-trans-retinoic acid, in which all double bonds on the chain part are in trans form, or 9-cis-retinoic acid, in which a double bond at the 9-position is cis form. Other retinoic acid isomers and retinoic acid derivatives can be also used in the present invention. As used herein, the above-mentioned retinoic acids, retinoic acid isomers and retinoic acid derivatives or their salts are collectively referred to as a retinoic acid. In the present invention, a retinoic acid used may be one kind of retinoic acid or a combination of plural kinds of retinoic acid.

A T cell is also called a T lymphocyte, and means a cell derived from a thymus among lymphocytes involved in an immunological response. The T cell includes a differentiated T cell and an undifferentiated T cell. Examples of known T cell include a helper T cell, a suppressor T cell, a killer T cell, a naive T cell, a memory T cell, an αβ T cell expressing TCRs of an α chain and α β chain, and a γδ T cell expressing TCRs of a γ chain and a δ chain. As used herein, examples of the "cell population containing a T cell or a precursor cell of a T cell" include, but not particularly limited to, a peripheral blood mononuclear cell (PBMC), a naive T cell, a hematopoietic stem cell, and an umbilical blood mononuclear cell. A variety of cell populations derived from hemocyte cells containing a T cell can be also used in the present invention. These cells may be activated in vivo or ex vivo by a cytokine such as IL-2. These cells may be collected from a living body or obtained through culturing ex vivo, and then may be used as they are or after freezing preservation. For example, cell populations obtained through various derivation operations or separation operations from cell populations obtained from a living body, for example, any cell populations obtained by separating cells such as a PBMC into CD8$^+$ (positive) or CD4$^+$ (positive) cells can be also used. Further, in the process for preparing a cell population of the present invention, materials containing the above-mentioned cells, for example, blood such as peripheral blood and umbilical blood, or materials obtained by removing components such as erythrocytes or plasma from blood, and bone marrow fluid can also be used.

A memory T cell is a specific type of a T cell capable of recognizing a foreign invader such as a bacterium or a virus which has been previously encountered via infection or vaccination. Upon a second encounter with an invader, the memory T cell initiates an immunological response faster and stronger than the time when the immune system first responded to the invader. The memory T cell includes two different cell populations of a central memory T cell and an effector memory T cell, based on difference in the homing ability or the effector function (Non-Patent Literature 2). The central memory T cell is believed to exhibit the property of a memory stem cell, and has the ability to self-replicate by high level phosphorylation of an important transcription factor known as STAT5. The central memory T cell is negative for CD45RA, but is positive for both CCR7 and L-selectin (CD62L). The effector memory T cell is negative for CCR7 and CD62L in addition to CD45RA.

The memory T cell is distinguished from a naive T cell by analysis of its cell surface antigen. The naive T cell is positive for cell surface antigen markers CD45RA, CCR7 and CD62L, while the memory T cell consisting of the central memory T cell and the effector memory T cell is negative for CD45RA. The memory T cell can be also distinguished from the naive T cell in that the memory T cell can rapidly initiate an immunological response as described above.

In the present invention, the "memory T-like cell" includes both a central memory T-like cell and an effector memory T-like cell.

In the present invention, the "preparation of a cell population containing a memory T-like cell" means a concept including induction of a memory T-like cell from a precursor cell having an ability to differentiate into a memory T-like cell, and proliferation (expansion culture) of the memory T-like cell. According to the present invention, a cell population containing a high proportion of a memory T-like cell can be obtained.

In the present invention, "fibronectin" or a fragment thereof may be an isolate from nature, an artificial preparation, or a recombinant prepared by genetic engineering, that is, a nonnatural product. Fibronectin or a fragment thereof can be produced in a substantially pure form from substances of the natural origin, for example, based on the disclosure of Ruoslahti E., et al., J. Biol. Chem., vol. 256, No. 14, pp. 7277-7281 (1981). In addition, a nucleic acid sequence encoding fibronectin and an amino acid sequence of fibronectin which are information for preparing a fibronectin fragment are disclosed in NCBI RefSeq Accession No. NM_002026 and NP_002017, respectively. As used herein, the substantially pure fibronectin or fibronectin fragment means that it does not essentially contain other proteins which exist with fibronectin in nature. The fibronectin or a fragment thereof can be used alone or as a mixture of plural kinds in the present invention.

A domain structure of fibronectin is divided into seven. The amino acid sequence of the domain structure includes three kinds of similar sequences, and the whole is comprised of repeats of these sequences. The three kinds of similar sequences are called type I, type II and type III, respectively. There are 14 type III sequences in fibronectin and, among them, the eighth, ninth and tenth sequences (hereinafter, referred to as III-8, III-9 and III-10, respectively) are contained in a cell-binding domain, and the twelfth, thirteenth and fourteenth sequences (hereinafter, referred to as III-12, III-13 and III-14, respectively) are contained in a heparin-binding domain. In addition, a region called IIICS exists at the C-terminal side of the heparin-binding domain. In IIICS, there is a region called CS-1 which consists of 25 amino acid residues and which has activity of binding with VLA-4. The fibronectin fragment which can be used in the present invention may be a fragment comprising any domain of III-7, 8, 9, 11, 12, 13 or CS-1 or, further, may be a fragment in which plural domains are repeatedly connected. Examples of the fibronectin fragment which can be used in the present invention include fragments containing a cell adhesion domain comprising a ligand to VLA-5, a heparin-binding domain, or a CS-1 domain which is a ligand to VLA-4. Examples of the recombinant fragment include CH-271 (SEQ ID No. 1 of Sequence Listing), CH-296 (SEQ ID No. 2 of Sequence Listing), H-271 (SEQ ID No. 3 of Sequence Listing), and H-296 (SEQ ID No. 4 of Sequence Listing) described in J. Biochem., vol. 110, pp. 284-291 (1991), or derivatives or modifications thereof. The CH-296 is commercially available under the name of RetroNectin (registered trademark, manufactured by TAKARA BIO INC.).

As used herein, an "active ingredient" refers to a retinoic acid, a CD3 ligand, fibronectin or a fragment thereof or a mixture thereof, other accessory stimulation factors, or suitable compounds, proteins, cytokines or other components which can be contained in media used in cell culture.

The present invention will be explained specifically below.

1. Process for preparing cell population containing memory T-like cell-1

The process for preparing a cell population containing a memory T-like cell of the present invention is a process comprising a step of ex vivo culturing a cell population containing a T cell using a retinoic acid and a CD3 ligand. In accordance with the present invention, it is possible to prepare ex vivo a cell population containing a memory T-like cell. Further, the memory T-like cell contained in the cell population obtained by the process has the ability to rapidly differentiate into a cell having cytotoxic activity (a cytotoxic lymphocyte) in response to even weak antigen stimulation, and is ssuitable for utilization in an immunotherapy etc.

In the process of the present invention, culture of the memory T-like cell is usually carried out in a medium containing predetermined components in the presence of active ingredients of the present invention. The cell number at initiation of culture used in the present invention is not particularly limited, and may be, for example, 10 to $1 \times 10^8$ cells/mL, preferably $10^2$ to $5 \times 10^7$ cells/mL, more preferably $10^3$ to $2 \times 10^7$ cells/mL.

The medium used in the process for preparing a cell population containing a memory T-like cell of the present invention is not particularly limited as long as it contains the active ingredients. The medium used can be a known medium prepared by mixing components suitable for maintenance and growth of a T cell or a precursor cell thereof and may be, for example, a commercially available medium or a modified medium thereof. The medium may contain suitable proteins, cytokines or other components in addition to their original constituent components. Suitably, in the process of the present invention, a medium containing IL-2 can be used. The concentration of IL-2 in the medium is not particularly limited, and is preferably 0.1 to $1 \times 10^5$ IU/mL, more preferably 1 to $1 \times 10^4$ IU/mL.

The concentration of a retinoic acid used in the present invention as the active ingredient in the medium is not particularly limited. When all-trans-retinoic acid is used, the concentration is, for example, preferably 0.01 to $10^4$ nM, more preferably 0.1 to $10^3$ nM. When 9-cis-retinoic acid is used, the concentration is, for example, preferably 0.1 to $10^3$ nM, more preferably 1 to $10^4$ nM.

In addition, the CD3 ligand used in the present invention as the active ingredient is not particularly limited as long as it is a substance having the activity of binding to CD3. Examples of the CD3 ligand include an anti-CD3 antibody, ConA, PHA, and PMA + ionomycin. Particularly preferably, an anti-CD3 monoclonal antibody, for example, OKT3 [Science, vol. 206, pp. 347-349 (1979)] is used in the present invention. The concentration of the CD3 ligand in the medium is not particularly limited. For example, when an anti-CD3 monoclonal antibody is used, the concentration is preferably 0.001 to 100 μg/mL, particularly preferably 0.01 to 100 μg/mL. For the purpose of activating a receptor on a lymphocyte, an anti-CD3 antibody may be added to the medium.

Further, fibronectin or a fragment thereof or a mixture thereof may be added as the active ingredient. The concentration of fibronectin or a fragment thereof or a mixture thereof during culture is not particularly limited, and is, for example, preferably 0.001 to 500 μg/mL, particularly preferably 0.01 to 500 μg/mL. In the present invention, culture in the presence of fibronectin or a fragment thereof or a mixture thereof may be carried out in only a part of the culturing step, or over the entire culturing step. In addition, a lymphocyte stimulation factor such as lectin can be also added to the medium. The concentration of the lymphocyte stimulation factor in the medium is not particularly limited as long as the desired effect is obtained.

In the present invention, if necessary, accessory stimulation can be also introduced by adding other accessory stimulation factors such as a CD28 ligand. Examples of other accessory stimulation factors include a desired antigen, a glucocorticoid-induced TNF-related receptor ligand (GITRL), an anti-CD28 antibody, an anti-CD80 antibody, and an anti-CD86 antibody.

Among these components, the "CD3 ligand" and the "fibronectin or a fragment thereof or a mixture thereof" may be dissolved in the medium to make them coexist or may be immobilized onto a suitable solid phase, for example, an instrument for cell culture (including an open system and a closed system) such as a petri dish, a flask or a bag, or a support for cell culture such as beads, a membrane or a glass slide, when they are used. The material of the solid phase is not particularly limited as long as it can be used for cell culture. For example, in the case where the components are immobilized onto the instrument, it is preferable that a certain amount of each component relative to the amount of the medium that will be put in the instrument is immobilized so that when the medium is put into the instrument, the ratio of the component to the medium is the same as the desired concentration for the case of dissolving the component in the medium. However, the amounts of immobilization of the components are not particularly limited as long as the desired effect is obtained. When the support is used, it is immersed in a culture liquid in an instrument for cell culture during cell culture. In the case where the components are immobilized onto the support, it is preferable that a certain amount of each component relative to the amount of the medium that will be put into the instrument is immobilized so that when the support is put into the medium, the ratio of the component to the medium is the same as the desired concentration for the case of dissolving the component in the medium. However, the immobilization amounts of the components are not particularly limited as long as the desired effect is obtained. In any case, immobilization of the component can be carried out according to a known method, for example, a method of immobilizing a fibronectin fragment described in WO97/18318 or WO00/09168. Further, an active ingredient other than the "CD3 ligand" and the "fibronectin or a fragment thereof or a mixture thereof" may be immobilized onto an instrument for cell culture or a support for cell culture.

If a component selected from the above-mentioned various components and the active ingredients of the present invention is immobilized onto a solid phase, the memory T-like cells can be easily separated from the active ingredients and the like by simply separating the memory T-like cells from the solid phase after the memory T-like cells are obtained by the process of the present invention, and thereby commingling of the active ingredients and the like with the memory T-like cells can be prevented.

The medium used in the present invention may contain cytokines, suitable proteins or other components in addition to the active ingredients. Examples of the cytokines include IL-7, IL-15, and IL-21. In addition, a lymphocyte stimulation factor such as lectin can also be added to the medium.

Although a medium not containing serum or plasma may be used in the present invention, serum or plasma may be added to the medium. The amount of serum or plasma to be added to the medium is not particularly limited, and the content of serum or plasma in the medium may be, for example, more than 0 to 20% by volume, preferably more than 0 to 5% by volume. The amount of serum or plasma to be used can be changed depending on the culturing stage. For example, serum or plasma can also be used while stepwisely decreasing the concentration thereof. The serum or plasma may be self-derived (meaning that the origin is the same as that of a cell to be cultured) or non-self-derived (meaning that the origin is different from that of a cell to be cultured). From the viewpoint of safety, self-derived serum or plasma is preferably used.

The cell number at initiation of culture used in the present invention is not particularly limited, and may be, for example, preferably 10 to $1 \times 10^8$ cells/mL, more preferably $10^2$ to $5 \times 10^7$ cells/mL, further preferably $10^3$ to $2 \times 10^7$ cells/mL. The culture conditions are not particularly limited, and conditions which are usually used for cell culture can be used. For example, cells can be cultured under the conditions of 37°C and 5% $CO_2$. An additional operation such as adding a fresh medium to a cell culture liquid at a suitable time interval to dilute the liquid, exchanging a medium, or exchanging a cell culture instrument may be carried out.

The cell culture instrument used in the process for preparing a cell population of the present invention is not particularly limited, and examples thereof include a petri dish, a flask, a bag, a large culture tank, and a bioreactor. As the bag, a $CO_2$ gas-permeable cell culture bag can be used. In the case of industrially preparing a large amount of a cell population, a large culture tank can be used. Although cell culture can be carried out in either an open system or a closed system, it is preferable to carry out the cell culture in a closed system from the viewpoint of safety of the obtained cell population.

A retinoic acid is preferably added to a cell culture liquid containing a T cell or a precursor cell of a T cell from the initiation of culture. For example, a culturing step in the presence of a retinoic acid and a CD3 ligand is carried out for at least 1 day or longer, more preferably 2 to 7 days, further preferably 2 to 5 days from the initiation of culture. Since there is a possibility that a retinoic acid is degraded in a culture liquid, a retinoic acid may be newly added at a suitable time interval.

The culture conditions are not particularly limited, and conditions which are usually used for cell culture can be used. For example, cells can be cultured under the conditions of 37°C and 5% $CO_2$. In addition, a medium can be exchanged with a fresh medium at a suitable time interval.

The cell population obtained by the process of the present invention contains a high proportion of cells which do not express CD45RA, but express CCR7 and/or CD62L, and are negative for both CCR7 and CD62L. All of CD45RA, CCR7 and CD62L are cell surface antigen markers of a lymphocyte. That is, cells contained in the cell population obtained by the process of the present invention at a high proportion can be classified into a memory T-like cell. As described in the Non-Patent Literatures 3 and 4, a memory T cell exhibits a high survival rate in a living body upon administration to the living body, a great cell proliferating effect, a great effect of accumulation into a tumor, and a high production rate of tumor-specific effector cells, and therefore it is useful in the field of cell therapy. In other words, the process of the present invention can increase the proportion of a memory T-like cell in a cell population. As used herein, an increase in the proportion of a memory T-like cell means that, when culture is carried out under the same conditions except for the presence or absence of the active ingredients of the present invention, the proportion of a memory T-like cell in a cell population obtained by carrying out the culture in the presence of the active ingredients used in the process of the present invention is higher as compared with the culture in the absence of the active ingredients. Preferably, a cell population having the proportion of a memory T-like cell which is 5% or more, more preferably 10% or more higher than that of a cell population obtained in the absence of the active ingredients can be obtained. In addition, the cell population obtained by the process of the present invention contains Tc2 type- and/or Th2 type-phenotype cells at a high proportion.

The cell population obtained by the process of the present invention may be further cultured in a medium containing or not containing a retinoic acid. Usually, the culture is carried out in the absence of a CD3 ligand.
In the present invention, the total number of days of culture is preferably 4 to 14 days, more preferably 5 to 14 days, further preferably 7 to 14 days.

In the cell population obtained by the process for preparing a cell population containing a memory T-like cell of the present invention, usually, cells other than the memory T-like cell are also present. In the present invention, cells are collected from the cell population by centrifugation or the like, and can be used as the memory T-like cell obtained by the process of the present invention, for example, as they are. If the active ingredients and the like are immobilized onto a cell culture instrument, commingling of the active ingredients and the like with the resulting memory T-like cell can be prevented.

The process of the present invention may further comprise a step of separating the memory T-like cell from the cell population. That is, in the present invention, a cell population in which the memory T-like cell is concentrated can be prepared for use by carrying out an operation of separating the cell population containing the memory T-like cell into cells other than the memory T-like cell and the memory T-like cell. For example, the process may comprise a step of removing CD45RA positive cells. Separation of the memory T-like cell can be carried out according to a known method. For example, the memory T-like cell can be separated by selectively collecting CD3 positive and CD45RA negative cells which can be distinguished using a flow cytometer by co-staining with a fluorescently labeled anti-CD3 antibody and a fluorescently labeled anti-CD45RA antibody. Alternatively, a cell population containing a high proportion of a memory T-like cell can be obtained by removing cells other than the memory T-like cell from the cell population obtained by the process of the present invention. The cell population containing a high proportion of a memory T-like cell according to the present invention also includes a cell population containing only the memory T-like cell.

According to the present invention, there is provided a process for preparing a cell population into which a desired gene is transferred utilizing the above-mentioned "process for preparing a cell population". The process comprises the following steps:

    (1) a step of ex vivo culturing a cell population containing a T cell or a precursor cell of a T cell, using a retinoic acid and a CD3 ligand, and
    (2) a step of transferring a desired gene into a cell population obtained in the step (1).

An efficiency of transferring a gene into a T cell is improved by carrying out a gene transfer operation after the step (1). That is, the cell population prepared by the process of the present invention is a cell population containing a high proportion of a T cell into which a desired gene is transferred. The present invention is particularly useful in preparation of cells for gene therapy.

The desired gene to be transferred into a T cell in the present invention is not particularly limited, and an arbitrary gene which is desired to be transferred into the cell can be selected from self-derived genes and foreign genes. Examples of such a gene include a gene encoding an antisense nucleic acid, a siRNA (small interfering RNA) or a ribozyme as well as a gene encoding a protein (e.g. an enzyme, a cytokine, or a receptor). In addition, a suitable marker gene which enables gene- transferred cells to be selected may be transferred together with the above-mentioned genes.

The desired gene to be transferred may be obtained from nature, or may be prepared by genetic engineering procedure, or may prepared by binding DNA molecules from different origins via a known means such as ligation. Further, the desired gene may have a sequence in which a mutation is introduced into the original sequence depending on the purpose.

According to the process of the present invention, for example, a gene encoding an enzyme associated with resistance to a drug used in treatment of a patient with cancer or the like can be transferred into a lymphocyte to confer the drug resistance to the lymphocyte. When the lymphocyte is used, an adoptive immunotherapy and a drug therapy can be combined, and thereby a higher therapeutic effect can be obtained. An example of the drug resistance gene is a multidrug resistance gene. On the other hand, contrary to the above-mentioned aspect, a gene conferring sensitivity to a specific drug can be transferred into a lymphocyte to confer sensitivity to the drug. In such a case, it becomes possible to remove a lymphocyte after transplantation into a living body by administering the drug. An example of the gene conferring sensitivity to a drug is a thymidine kinase gene.

One aspect of the present invention is exemplified by transfer of a gene encoding a receptor which recognizes a desired antigen, without particular limitation. Examples of the gene include a gene encoding a T cell receptor (TCR) which recognizes a surface antigen of a target cell, and a gene which has an antigen recognition site of an antibody to a surface antigen of a target cell and encodes a chimeric receptor comprising an intracellular region of a TCR. A cell population containing a T cell into which the gene is transferred is a cell population containing a T cell which recognizes the desired antigen. Since the cell population has higher specificity for a desired antigen as compared with a cell population into which a gene encoding a receptor is not transferred, and can specifically react with the desired antigen in response to stimulation by the desired antigen, it is useful for utilization in immunotherapy.

In the process for preparing a cell population into which a gene is transferred of the present invention, the first step is carried out according to the above-mentioned "Process for preparing cell population containing a memory T-like cell". After culture of the first step is carried out at least for 1 day or longer, more preferably for 2 to 7 days, further preferably for 2 to 5 days, the second step described later is carried out.

In the process for preparing a cell population into which a gene is transferred of the present invention, the resulting cell population is a cell population containing a high proportion of Tc2-type and/or Th2-type cells, in which the proportion of a memory T-like cell is increased, the proportion of a cell antigen-specifically producing IFN-γ is reduced and the proportion of a cell producing IL-4 and perforin is improved. Since the cell population is useful for reducing GVHD and potentiating GVL in an immunological response to parasite infection and organ transplantation through potentiation of humoral immunity, it is extremely suitable for use in the field of cell therapy. Particularly, the cell population is useful in a donor lymphocyte infusion therapy for relapsed leukemia after homogeneous hematopoietic stem cell transplantation, because it is believed that the rate of causing graft versus host disease (GVHD) as a side effect is lower as compared with Tc1 cells [Fowler D. H. and one other person, Leukemia and Lymphoma, vol. 38, pp. 221-234 (2000)].

In the process for preparing a cell population of the present invention, the second step is a step of transferring a desired gene into the cell obtained in the first step. The number of the desired gene to be transferred into the cell is not limited, and one gene or plural genes (e.g. 1 to 9 genes) may be transferred. For example, a suitable gene such as a gene encoding a T cell surface antigen can be transferred at the same time, in advance, or afterwards, depending on the cell into which the gene is transferred. For example, in the case where an αβ TCR gene is transferred into a γδ T cell, it is preferable that a gene encoding CD8 is transferred simultaneously.

In the present invention, a means for transferring a desired gene is not particularly limited, and a suitable means selected from known gene introduction methods can be used. As the gene transfer method, either a method using a virus vector or a method not using the vector can be used in the present invention. With respect to details of these methods, many literatures have been already published.

The virus vector is not particularly limited, and a known virus vector which is usually used in a gene transfer method, for example, a retrovirus vector (including a lentivirus vector and a pseudotyped vector), an adenovirus vector, an adeno-associated virus vector, a simian virus vector, a vaccinia virus vector, a sendaivirus vector or the like can be used. Particularly preferably, a retrovirus vector, an adenovirus vector or a lentivirus vector is used. As the virus vector, preferred is a virus vector lacking the replication ability so as not to self-replicate in an infected cell.

Examples of the gene transfer method not using a virus vector which can be used in the present invention include, but not limited to, a method using a carrier such as liposome or ligand-polylysine, a calcium phosphate method, an electroporation method, and a particle gun method. In this case, a foreign gene incorporated into a plasmid DNA or a straight DNA or RNA is transferred.

A retrovirus vector and a lentivirus vector can stably integrate a foreign gene inserted in the vector into the chromosomal DNA of a cell into which the vector is transferred, and they are used for the purpose of gene therapy or the like. Since the vectors can infect cells undergoing division or growth, they are particularly preferably used for performing gene transfer in the process of the present invention.

For example, a desired gene can be inserted into a vector, a plasmid or the like so as to express the gene under the control of a suitable promoter. In addition, in order to attain efficient transcription of the gene, another regulatory element that cooperates with a promoter or a transcription initiation site, for example, an enhancer sequence or a terminator sequence may be present in the vector. In addition, for the purpose of insertion by homologous recombination of the desired gene into the chromosome of a target T cell, for example, the gene may be placed between flanking sequences comprising nucleotide sequences, each having homology with nucleotide sequences present on the both sides of a desired target insertion site of the gene in the chromosome.

Examples of the vector that can be used in the present invention include retrovirus vectors such as a MFG vector, an α-SGC vector (WO92/07943), pBabe [Morgenstern J. P., Land H., Nucleic Acids Research, vol. 18, No. 12, pp. 3587-3596 (1990)], pLXIN (manufactured by Clontech), and pDON-AI (manufactured by TAKARA BIO INC.), lentivirus vectors [a human immunodeficiency virus (HIV)-derived vector and a simian immunodeficiency virus (SIV)-derived vector], and vectors obtained by modifying them.

In addition, these vectors can be prepared as virus particles in which the vectors are packaged, by using known packaging cell lines, for example, PG13 (ATCC CRL-10686), PG13/LNc8 (ATCC CRL-10685), PA317 (ATCC CRL-9078), GP+E-86 (ATCC CRL-9642), GP+envAm12 (ATCC CRL-9641), and ψCRIP described in Proceedings of the National Academy of Sciences of the USA, vol. 85, pp. 6460-6464 (1988). In addition, retrovirus-producer cells can be also prepared using a 293 cell or a 293 T cell having a high transfection efficiency.

In the present invention, a retrovirus prepared by pseudotyped packaging which has an envelope derived from a different virus from a virus from which the genome of the retrovirus is derived, can be also used. For example, a pseudotyped retrovirus having an envelope derived from a molony mouse leukemia virus (MoMLV), a gibbon ape leukemia virus (GaLV), a vesicular stomatitis virus (VSV) or a feline endogenous virus, or a protein capable of functioning as an envelope can be used. Further, a retrovirus having, on a surface thereof, a sugar chain-modified protein prepared by using a retrovirus-producer cell into which a gene of an enzyme involved in sugar chain synthesis or the like is transferred can be used also in the present invention. The above-mentioned virus can be prepared using a packaging cell expressing each envelope. As the packaging cell, a variety of packaging cells have been already reported, and some of them are commercially available. In the present invention, these known packaging cells can be used.

When gene transfer is carried out using a retrovirus vector, a functional substance having retrovirus-binding activity can be used to improve a gene transfer efficiency. Examples of the functional substance having retrovirus-binding activity used in the process include, but not particularly limited to, a heparin-II-binding region of fibronectin, a fibroblast growth factor, V-type collagen, fragments of the above-mentioned polypeptides, polylysine, and DEAE-dextran. It is preferable that the fibronectin fragment has a heparin-II-binding region in the molecule, and such a fragment is also described in WO95/26200 and WO97/18318. CH-296, which is a fibronectin fragment having a heparin-II-binding region, is commercially available under the name of RetroNectin (registered trademark, manufactured by TAKARA BIO INC.). In addition, substances functionally equal to these functional substances, for example, a functional substance having a heparin-binding site can be also used. In addition, a mixture of the functional substances, a polypeptide containing the functional substance, a polymer of the functional substance, a derivative of the functional substance and the like can be also used.

In addition, a functional substance having target cell-binding activity may be used together in the present invention. The substance is useful for improving a gene transfer efficiency into a target cell or performing target cell-specific gene transfer. An example of the functional substance having target cell-binding activity is, but not particularly limited to, a substance having a ligand capable of binding to a target cell. Examples of the ligand include a cell-adherent protein (fibronectin, laminine, collagen and the like) or a fragment thereof, a hormone, a cytokine, an antibody to an antigen on the cell surface, a polysaccharide, a glycoprotein, a glycolipid, a sugar chain derived from a glycoprotein or a glycolipid, and metabolites of a target cell. In addition, a polypeptide containing the functional substance, a polymer of the functional substance, a derivative of the functional substance, and a functionally equivalent substance of the functional substance can also be used. The functional substance having target cell-binding activity may be immobilized onto a solid phase, like the functional substance having retrovirus-binding activity. As the functional substance having retrovirus-binding activity, a substance having target cell-binding activity in addition to retrovirus-binding activity can be also used.

As explained above, according to the process of the present invention comprising gene transfer after culture in the presence of the active ingredients including a retinoic acid, it becomes possible to efficiently perform gene transfer into a cell population containing a T cell. In addition, the process of the present invention does not require a special facility or apparatus. Many kinds of retrovirus vectors and target cells are effective in the process of the present invention. Further, since the process of the present invention is suitable for utilization in a closed system, it is very clinically useful for gene therapy and the like.

The process for preparing a cell population of the present invention may further include, as the third step, a step of culturing the gene-transferred cell obtained in the second step.
The above-mentioned step may be carried out by a usual cell culture method, for example, a known T cell culture method. Cell culture may be carried out under the same conditions as those of the first step, or under the same conditions as used in the first step except that any one or both of a retinoic acid and a CD3 ligand used in the first step as the active ingredients are not used.

In the process for preparing a cell population of the present invention, the total number of days of culture is preferably 4 to 14 days. When the total number of days of culture is less than 4 days, satisfactory cell number for use in general immunotherapy cannot be obtained. When the total number of days of culture exceeds 14 days, the cell growth rate is reduced. In the present invention, the total number of days of culture is more preferably 5 to 14 days, further preferably 7 to 14 days.

2. Process for preparing cell population containing memory T-like cell-2

The present inventors surprisingly found that, a change in the timing of adding a retinoic acid in the above-mentioned "Process for preparing cell population containing memory T-like cell-1" resulted in a cell population containing a high proportion of Tc1-type and/or Th1-type cells can be prepared instead of a cell population containing a high proportion of Tc2-type and/or Th2-type cells. The process includes the following steps.

  (1) a step of ex vivo culturing a cell population containing a T cell or a precursor cell of a T cell in the presence of a CD3 ligand and in the absence of a retinoic acid, and
  (2) a step of ex vivo culturing the cell population obtained in the step (1) in the presence of a retinoic acid.

In the process, the first step is a step of ex vivo culturing a cell population containing a T cell or a precursor cell of a T cell in the presence of a CD3 ligand, and is carried out according to the above-mentioned "Process for preparing cell population containing memory T-like cell-1" expect for use of a retinoic acid used as the active ingredient. After the culture of the first step is carried out at least for 1 day or longer, more preferably for 2 to 7 days, further preferably for 2 to 5 days, the second step described later, that is, a step of ex vivo culturing a cell population obtained in the first step in the presence of a retinoic acid is carried out. The culture of the second step is carried out at least for 1 day or longer, more preferably for 2 to 10 days, further preferably for 3 to 9 days.
The cell population obtained by the process is a cell population containing a high proportion of Tc1 type and/or Th1 type cells, in which the proportion of a memory T-like cell is increased, the proportion of a cell antigen-specifically producing IL-4 is reduced and the proportion of a cell producing IFN-$\gamma$ and perforin is improved. Since the cell population has strong cytotoxic activity on a tumor and activates cellular immunity, it is extremely suitable for use in the filed of cell therapy.
The second step may be carried out by a usual cell culture method, for example a known T cell culture method, except that it is carried out in the presence of a retinoic acid. The cell culture may be carried out under the same conditions as those described in the above-mentioned "Process for preparing cell population containing memory T-like cell-1" or under the same conditions as used in the above-mentioned process except that a CD3 ligand is not used. In addition, since there is a possibility that a retinoic acid is degraded in a culture liquid, a retinoic acid may be newly added at a suitable time interval, or a medium may be exchanged with a fresh medium at a suitable time interval.
Also in the process, preparation of a gene-transferred cell can be carried out by adding an operation of gene transfer at a suitable stage during the process. The operation of gene transfer can be carried out at an arbitrary stage as long as cell culture is not affected. For example, an operation of gene transfer may be carried out between the first step and the second step. After transfer of a gene into the cell population obtained in the first step is carried out, culture may be continued in the same medium for 1 to 2 days followed by addition of a retinoic acid to the medium. A method of the gene transfer is not particularly limited, and the same method as that described with respect to the above-mentioned "Process for preparing cell population containing memory T-like cell-1" can be used.
It is preferable that in the process, the total number of days of culture is suitably 4 to 14 days. When the total number of days of culture is less than 4 days, satisfactory cell number for use in general immunotherapy cannot be obtained. When the total number of days of culture exceeds 14 days, the cell growth rate is reduced. In the present invention, the total number of days of culture is more preferably 5 to 14 days, further preferably 7 to 14 days.
As described in the following Examples, the cell population prepared by the process contains a high proportion of a Tc1-type and/or Th1-type memory T-like cell. Since the Tc1-type or Th1-type cell population exerts an action via potentiation of cellular immunity, it is useful in an immunological response to an intracellular parasite and tumor immunity. Further, as described in Example 6, expression of a transferred gene is increased by adding a retinoic acid after gene transfer.
As described above, according to the present invention, there is provided a process for preparing a cell population containing a Tc2 and/or Th2-type or Tc1 and/or Th1-type memory T-like cell, which comprises carrying out culture of a cell population containing a T cell or a precursor cell of a T cell by using a CD3 ligand and a retinoic acid and controlling the timing of addition of a retinoic acid. Further, the present invention can also be utilized for preparing a cell population containing a Tc2 and/or Th2-type or Tc1 and/or Th1-type memory T-like cell into which a desired gene is transferred.
Further, the process of the present invention can also include a step of separating a cell population into arbitrary cell sub-populations, before or after each step. For example, since a memory T-like cell is contained at a high proportion in the cell population obtained by culturing in the presence of the active ingredients of the present invention as described above, the cell population can be further separated to obtain a cell expressing a desired surface antigen marker, and thereby a memory T-like cell or a T cell population containing a high proportion of a memory T-like cell can be obtained.

In the case of preparation of a cell population into which a gene is transferred, cells expressing the desired gene may be separated and obtained during or after completion of the process of the present invention. A separation operation is not particularly limited, and for example, the cells can be separated by a known procedure using a cell sorter, magnetic beads, a column or the like. If there is a suitable means, a cell sub-population consisting of cells expressing an transferred gene may be selected.

It is also possible to stably maintain a T cell by cloning a T cell from the cell population prepared by the process of the present invention. In addition, the cell population obtained by the process of the present invention can be further cultured by the process of the present invention or a known process, and thereby a new cell population can be obtained.

The process of the present invention is a novel method of expansion-culturing a T cell for adoptive immunotherapy which comprises a step of stimulation with a retinoic acid and a CD3 ligand and, optionally, a step of transfer of a desired gene. The cell population obtained by the process of the present invention can be effectively transplanted into a living body and retained therein. That is, the process of the present invention can provide a more useful cell population for cell therapy, which can be maintained in a living body into which the cell population is transplanted at a high concentration over a long term, and can be widely applied to all gene therapies based on a T cell.

As used herein, the process for preparing a cell or a cell population comprises a step of culturing a cell or a cell population, and refers to a method which can further include steps of inducing (activating), expansion culturing and separating the cell or the cell population, or a step of a combination of them.

3. Cell population, medicament, therapeutic method or prophylactic method, or use of the present invention

The cell population prepared by the process of the present invention is suitable for use in immunotherapy, as described above. The gene-transferred cell population prepared by the process of the present invention is a cell population having the desired effect in accordance with the transferred desired gene. For example, when a gene encoding a receptor which recognizes a desired antigen is transferred, a cell population into which the gene is transferred can exhibit cytotoxic activity on a cell presenting an antigen recognized by the receptor encoded by the transferred receptor gene, and therefore it is useful in treatment of a variety of diseases. Examples of a disease for which the cell population is administered include, but not particularly limited to, cancer (leukemia, a solid tumor, and the like), hepatitis, autoimmune diseases, influenza, and infectious diseases caused by a virus such as HIV, a bacterium or a fungus (e.g. tuberculosis, MRSA infectious disease, VRE infectious disease, and deep mycosis). In addition, the cell population prepared by the process of the present invention can be also utilized for the prevention of infectious disease after bone marrow transplantation or irradiation irradiation, donor lymphocyte infusion for the purpose of remission of relapsed leukemia, immunotherapy, adoptive immunotherapy, or the like.

Further, the present invention provides a medicament (therapeutic agent) for the above-mentioned diseases which contains the cell population obtained by the process of the present invention as an active ingredient. The therapeutic agent containing the cell population is particularly suitable for use in immunotherapy. In immunotherapy, a T cell suitable for treatment of a patient is administered intravenously, intraarterially, subcutaneously or intraperitoneally, for example, by injection or drip infusion. The therapeutic agent is very useful in use for the above-mentioned diseases or donor lymphocyte infusion. The therapeutic agent can be prepared as a drip infusion agent or an injectable, for example, by mixing the T cell population prepared by the process of the present invention as an active ingredient with a known organic or inorganic carrier, excipient, stabilizer and the like, which are suitable for parenteral administration, according to a method known in the pharmacy field. Various conditions for the therapeutic agent such as the content of the cell population of the present invention in the therapeutic agent and the dose of the therapeutic agent can be appropriately determined according to a known immunotherapy. For example, the content of the T cell population of the present invention in a medicament is not particularly limited, and may be, for example, preferably $1 \times 10^3$ to $1 \times 10^{11}$ cells/mL, more preferably $1 \times 10^4$ to $1 \times 10^{10}$ cells/mL, further preferably $1 \times 10^3$ to $1 \times 10^9$ cells/mL. The dose of the medicament of the present invention is not particularly limited, and may be, for example, preferably $1 \times 10^6$ to $1 \times 10^{12}$ cells/day, more preferably $1 \times 10^7$ to $5 \times 10^{11}$ cells/day, further preferably $1 \times 10^8$ to $2 \times 10^{11}$ cells/day for adult. An immunotherapy with the therapeutic agent can be also used in combination with a drug therapy by administration of a known drug, a radiation therapy or a therapy by a surgical operation.

The present invention also provides a method of treating or preventing the above-mentioned diseases, which comprises administering an effective amount of the cell population obtained by the above-mentioned process to a subject. As used herein, the subject is not particularly limited, and preferably refers to a living body (e.g. a human patient or a non-human animal) having the above-mentioned diseases for which the T cell population prepared by the process of the present invention is administered. The therapeutic agent of the present invention containing, as an active ingredient, the cell population into which a gene encoding a T cell receptor is transferred can be administered to a subject expressing an HLA molecule which is the same as or has up to 3 gene locus mismatches with the HLA molecule expressed by the cell population.

As used herein, the effective amount is the amount of the T cell population exerting a therapeutic or prophylactic effect when the T cell population is administered to the subject, as compared with a subject to which the T cell population is not administered. The specific effective amount can vary depending on the dosage form, administration method, purpose of use, age, body weight or symptom of the subject, and the like, and it is preferably similar to the case of the above-mentioned medicament. An administration method is not limited, and for example, the effective amount may be administered by drip infusion, injection or the like, like in the case of the above-mentioned medicament.

Further, the present invention provides a diagnostic agent containing the cell population as an active ingredient. For example, a cell obtained from a patient is analyzed and screened with the diagnostic agent of the present invention, and thereby presumption of a therapeutic effect, selection of an effective transfer gene, or the like is possible.

In addition, the present invention can prepare a cell population containing an activated T cell by giving stimulation to the cell population obtained by the above-mentioned process of the present invention with at least one stimulation factor selected from the group consisting of an antigen, a CD3 ligand, a CD28 ligand, a cytokine, a chemokine, and a cell having the ability to produce a cytokine. Further, the present invention provides the cell population obtained by the process. The thus obtained cell population containing an activated T cell can be used as an active ingredient of a medicament, like the T cell population obtained by the above-mentioned process. As used herein, the stimulation with a stimulation factor is not particularly limited as long as it can activate the cell population obtained by the above-mentioned process of the present invention. For example, culture is carried out under the coexistence of the T cell population obtained by the process of the present invention and the stimulation factor.

As used herein, the antigen to be presented is not particularly limited as long as a peptide is presented on a cell expressing a MHC molecule or the antigen can be recognized by a T cell to efficiently activate the T cell, and examples thereof include a peptide, a glycopeptide, a tumor cell extract, a tumor cell-sonicated product, a tumor cell hot water-treated product, a nucleic acid (a DNA or an RNA) of a virus or the like, a bacterium, and a protein. Examples of the CD3 ligand and the CD28 ligand are the same as the above-mentioned examples of the CD3 ligand and the CD28 ligand.

As used herein, the cytokine is not particularly limited as long as it can act on and activate a T cell, and examples thereof include IL-2, IFN-γ, TGF-β, IL-15, IL-7, IFN-α, IL-12, CL40L, and IL-27. The chemokine is not particularly limited as long as it acts on a T cell and exhibits migration activity, and examples thereof include RANTES, CCL21, MIP1α, MIP1β, CCL19, CXCL12, IP-10, and MIG.

As used herein, the cell having the ability to produce a cytokine is not particularly limited as long as it has the ability to produce the above-mentioned cytokine.

An activated lymphocyte population having the cytokine producing ability can be produced by giving costimulation with an anti-CD3 antibody and an anti-CD28 antibody to the cell population obtained by the process of the present invention.

A useful antigen-specific CTL which has extremely high cytotoxic activity and has high antigen recognizing ability can be induced by giving antigen stimulation to the T cell population obtained by the process of the present invention. The culture can be carried out in the presence of the above-mentioned fibronectin or a fragment thereof or a mixture thereof, or a known component used in culturing a T cell, in addition to the above-mentioned stimulation factor. The T cell population thus prepared is an extremely useful T cell population which has high treating effect in a living body over a long term.

The present invention also provides use of the cell population obtained by the above-mentioned process for production of a medicament. A process for preparing the medicament is carried out similarly to the case of the above-mentioned medicament. A disease for which the medicament is administered is not particularly limited, and examples thereof are the same as those for the above-mentioned medicament.

Examples

The present invention will be explained further specifically below by way of examples, but the present invention is not limited only to the following examples.

Among the operations described herein, basic operations were carried out according to the methods described in Molecular Cloning: A Laboratory Manual 3rd ed., edited by T. Maniatis et al., published by Cold Spring Harbor Laboratory in 2001.

Example 1 Culture in the presence of retinoic acid

A fibronectin fragment [RetroNectin (registered trademark), manufactured by TAKARA BIO INC.] and an anti-CD3 antibody (OKT3, Janssen Pharmaceutical K.K.) were dissolved in PBS at each concentration of 5 μg/mL. This solution was added to a surface-untreated 12-well plate in an amount of 1 mL/well, and allowed to stand at 37°C for 5 hours. Similarly, an anti-CD3 antibody alone was dissolved in PBS to 5 μg/mL, added to a tissue culture-untreated 12-well plate in an amount of 1 mL/well, and then allowed to stand at 37°C for 5 hours. Then, each solution was removed, and each plate was washed twice using 2 mL/well of PBS to prepare a fibronectin fragment/anti-CD3 antibody-adherent plate and an anti-CD3 antibody-adherent plate.

A human peripheral blood mononuclear cell (PBMC) prepared from a healthy person from whom informed consent had been obtained was suspended at $0.2 \times 10^6$ cells/mL in a GT-T503 medium (manufactured by TAKARA BIO INC.) containing 1% autologous plasma, 720 IU/mL IL-2, 0.2% human serum albumin (HSA), 2.5 μg/mL Fungizone (manufactured by Bristol-Myers Squibb) and 10 nM retinoic acid (manufactured by Wako Pure Chemical Industries, Ltd.). The suspension was added to the fibronectin fragment/anti-CD3 antibody-adherent plate or the anti-CD3 antibody-adherent plate at 2.6 mL/well. As a control, a cell suspension not containing a retinoic acid was prepared, and added to the plates similarly. Each plate was cultured at 37°C for 4 days in a $CO_2$ incubator.

After 4 days from culturing, cells were recovered, and cell surface markers were measured.

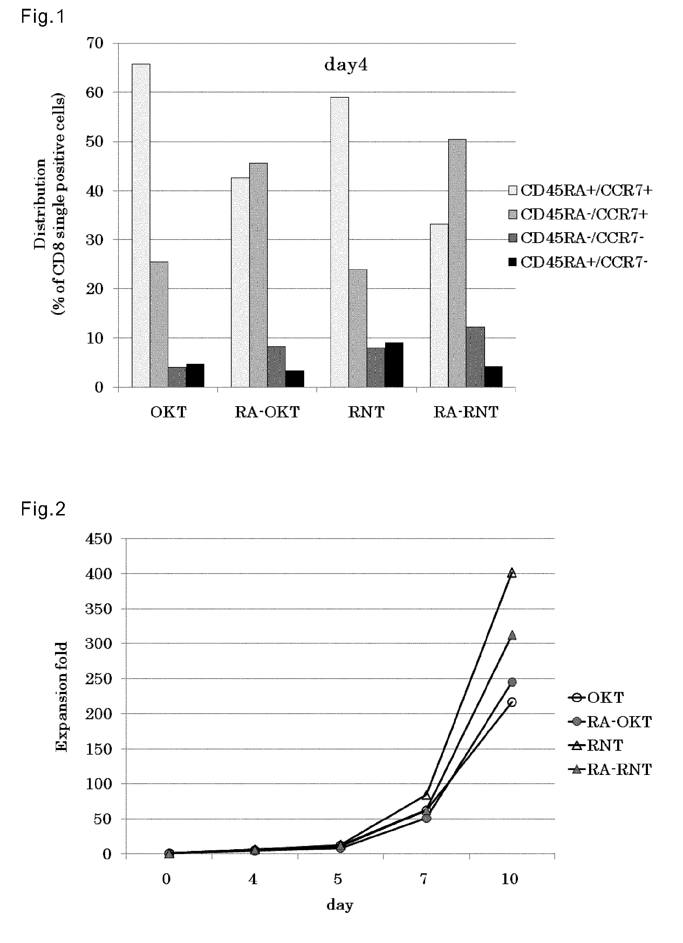

The results of measurement of the cell surface markers are shown in Fig. 1. The proportion of memory T cells in CD8 single positive cells in cultured cells was measured by staining the cells with an anti-CD8 antibody, an anti-CD45RA antibody and an anti-CCR7 antibody. Regarding the cell surface markers, a naive T-like cell is CD45RA+/CCR7+, a central memory T-like cell is CD45RA-/CCR7+, and an effector memory T-like cell is CD45RA-/CCR7-. Further, an effector T-like cell is CD45RA+/CCR7-. In each stimulation condition of Fig. 1, bars represent the proportions (%) of CD45RA+/CCR7+, CD45RA-/CCR7+, CD45RA-/CCR7- and CD45RA+/CCR7- cells in CD8 single positive cells from left. In each graph of Figs. 1 to 15, OKT indicates stimulation by an anti-CD3 antibody alone, RA-OKT indicates retinoic acid/anti-CD3 antibody stimulation, RNT indicates fibronectin fragment/anti-CD3 antibody stimulation, and RA-RNT indicates retinoic acid/fibronectin fragment/anti-CD3 antibody stimulation.

As seen from Fig. 1, the proportions of central memory T-like cells and effector memory T-like cells were increased by a combination of both stimulation by an anti-CD3 antibody alone or fibronectin fragment/anti-CD3 antibody stimulation with retinoic acid stimulation.

Example 2 TCR gene-transferred cell

In the same way as in Example 1-(1) to (4) of WO2008/11150 a series of operations of cloning of a MAGE-A4-specific TCR gene, construction of a vector and preparation of a producer cell were carried out to prepare a MS-bPa virus liquid. The RNA copy number of the virus liquid was $2.55 \times 10^{11}$ copies/mL.

(1) Preparation of TCR gene-transferred cell

A fibronectin fragment and an anti-CD3 antibody were dissolved in PBS at 25 μg/mL and 5 μg/mL respectively. This solution was added to a tissue culture-treated 12-well plate in an amount of 0.4 mL/well, and allowed to stand at 37°C for 5 hours. Similarly, an anti-CD3 antibody (OKT3, Janssen Pharmaceutical K.K.) was dissolved in PBS at 5 μg/mL, and this solution was added to a tissue culture-treated 12-well plate in an amount of 0.4 mL/well, and allowed to stand at 37°C for 5 hours. Then, each solution was removed, and each plate was washed twice using 2 mL/well of a GT-T503 medium (manufactured by TAKARA BIO INC.) to prepare a fibronectin fragment/anti-CD3 antibody-adherent plate or an anti-CD3 antibody-adherent plate.

A PBMC prepared from a healthy person from whom informed consent had been obtained was suspended at $0.18 \times 10^6$ cells/mL in a GT-T503 medium containing 1% autologous plasma, 720 IU/mL IL-2, 0.2% HSA, 2.5 μg/mL Fungizone and 10 nM retinoic acid. The suspension was added to the fibronectin fragment/anti-CD3 antibody-adherent plate or the anti-CD3 antibody-adherent plate at 2.65 mL/well. As controls, a cell suspension not containing retinoic acid was added to the plates. Each plate was cultured at 37°C for 4 days in a $CO_2$ incubator.

A virus-bound plate for gene transfer was prepared as follows. A fibronectin fragment was dissolved in PBS at 20 μg/mL, and this solution was added to a tissue culture-untreated 24-well plate in an amount of 0.5 mL/well, and allowed to stand at 4°C for 3 days. Then, the solution was removed, and the plate was washed twice using 1 mL/well of PBS. To the wells, 0.9 mL of the MS-bPa virus liquid was added. This plate was centrifuged at 32°C and $2000 \times g$ for 2 hours. After centrifugation, the virus liquid was removed, and the plate was washed three times using 1 mL/well of 1.5% HSA-containing-PBS to prepare a virus-bound plate.

A gene transfer operation was carried out as follows. The PBMC which had been stimulated with an anti-CD3 antibody alone or a fibronectin fragment/an anti-CD3 antibody for 4 days was added to the virus-bound plate at $0.8 \times 10^6$ cells/2.0 mL/well. This plate was cultured at 37°C overnight in a $CO_2$ incubator. The cultured cells were suspended in a GT-T-RetroIII medium (manufactured by TAKARA BIO INC.) containing 5 mM sodium butyrate (manufactured by Wako Pure Chemical Industries, Ltd.) at $0.68 \times 10^6$ cells/1.9 mL/well, seeded on a fresh virus-bound plate, and cultured at 37°C for 4 hours in a $CO_2$ incubator. After completion of culture, the cells were recovered, suspended in a GT-T 503 medium containing 1% autologous plasma, 720 IU/mL IL-2, 0.2% HSA, 2.5 μg/mL Fungizone and 10 nM retinoic acid at $0.1 \times 10^6$ cells /mL, seeded again on a 12-well plate for cell culture and then, cultured at 37°C in a $CO_2$ incubator. As a control, a gene transfer operation and cell culture after gene transfer were similarly carried out using a cell suspension not containing retinoic acid.

On the 7th day after initiation of stimulation of the PBMC, the cell was seeded at $0.1 \times 10^6$ cells/mL, and cultured until the 10th day. The cell growth rate was measured on the 4th, 5th, 7th and 10th days. On the 4th day, cell surface markers before the cell was transferred to the virus-bound plate were measured. On the 10th day, cell surface markers, expression of a transferred TCR and the copy number of a transferred gene were measured.

The results of measurement of the cell growth rate are shown in Fig. 2. In Fig. 2, the ordinate axis indicates the growth rate (%) relative to the cell number at initiation of culture, and the abscissa axis indicates the number of days of culture. As seen from Fig. 2, the growth rates of fibronectin fragment/anti-CD3 antibody-stimulated cells were higher, as compared with stimulation by an anti-CD3 antibody alone, regardless of the presence or absence of retinoic acid stimulation.

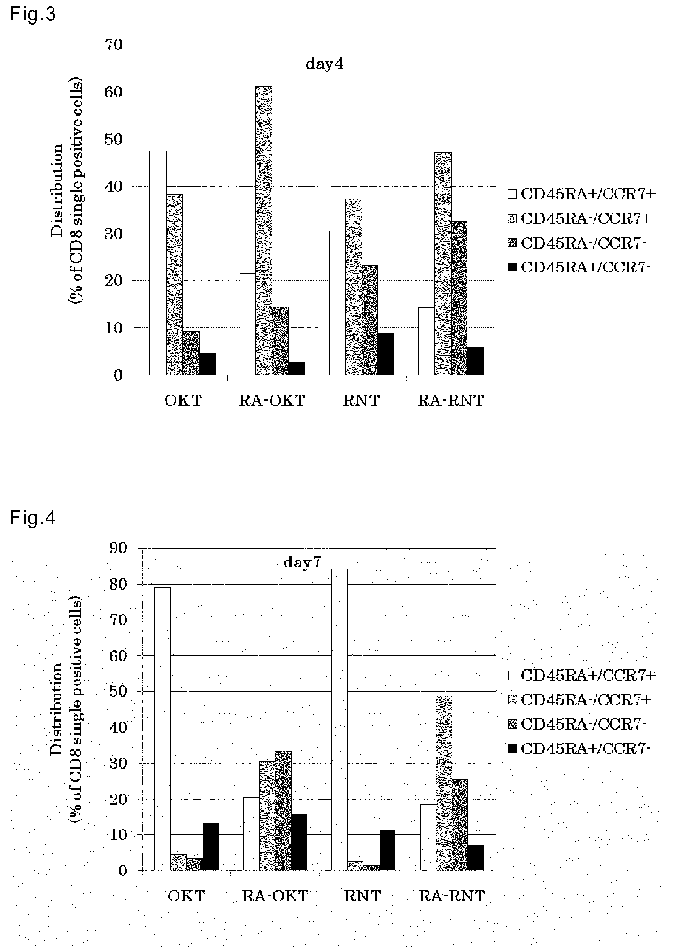

The results of measurement of cell surface markers of CD8 single positive cells in cultured cells on the 4th day after initiation of stimulation of the PBMC are shown in Fig. 3, and the results of measurement of cell surface markers of CD8 single positive cells in cultured cells on the 7th day are shown in Fig. 4. The proportion of memory T cells was measured by staining the cells with an anti-CD8 antibody, an anti-CD45RA antibody and an anti-CCR7 antibody. Under each stimulation condition in Fig. 3 and Fig. 4, bars represent the proportions (%) of CD45RA+/CCR7+, CD45RA/CCR7+, CD45RA-/CCR7- and CD45RA+/CCR7- cells in CD8 positive cells from left. As seen from Fig. 3 and Fig. 4, the proportions of central memory T-like cells and effector memory T-like cells were increased by a combination of both stimulation by an anti-CD3 antibody alone or fibronectin fragment/anti-CD3 antibody stimulation with retinoic acid stimulation.

A gene transfer efficiency was determined by measuring expression of a MAGE-A4-specific TCR by a tetramer assay.

According to the description of WO2008/11150 a polypeptide in which a sequence of a substrate for a biotin protein ligase BirA is added to the C-terminal of an HLA-A2402 heavy chain, and β2-microglobulin were expressed in Escherichia coli as an insoluble inclusion body. The inclusion body was refolded in vitro in the presence of a P143 peptide to form an HLA-A2402/β2-microglobulin/P143 complex. The resulting complex was treated with a biotin protein ligase (manufactured by Avidity) and a tetramer was prepared using phycoerythrin-labeled streptavidin (Straptavidin-PE, manufactured by Invitrogen).

After initiation of stimulation of the PBMC, the cells on the 10th day were mixed with the tetramer to react at 37°C for 30 minutes and then reacted with an APC-Cy7-labeled anti-human CD8 antibody (manufactured by Becton Dickinson) at 4°C for 20 minutes. After completion of the reaction, the cells were washed, and then subjected to flow cytometry analysis using FACScant (manufactured by Becton Dickinson).

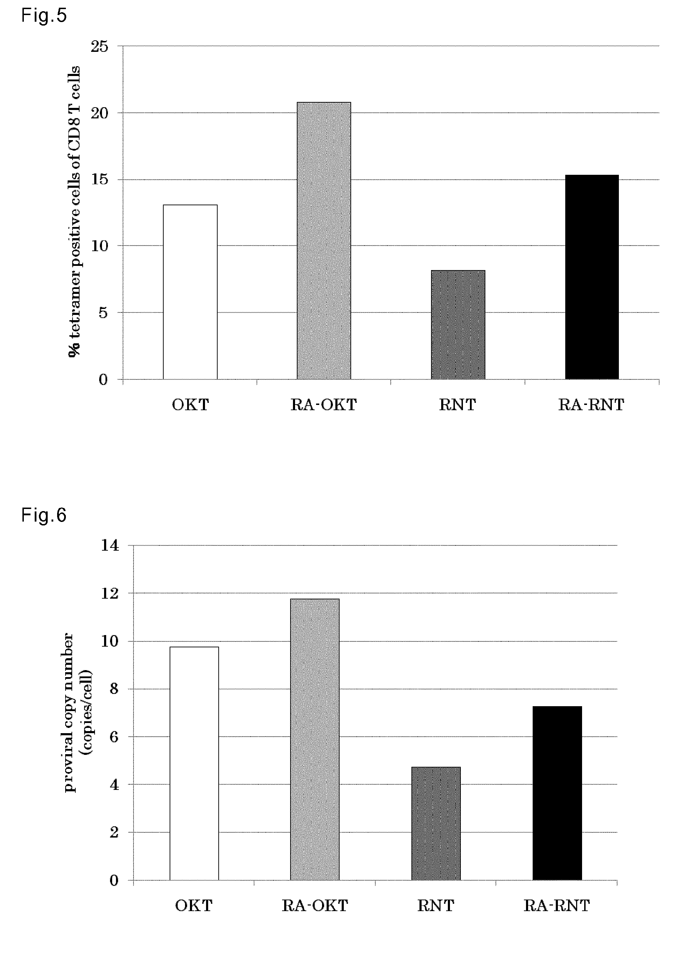

The results of tetramer measurement are shown in Fig. 5. The ordinate axis indicates the proportion (%) of tetramer positive cells in CD8 single positive cells. As a result, the proportion of a MAGE-A4-specific TCR-expressing cells was increased by a combination of stimulation by an anti-CD3 antibody alone or fibronectin fragment/anti-CD3 antibody stimulation with retinoic acid stimulation.

The provirus copy number of the transferred gene in the gene-transferred cell was measured with Provirus Copy Number Detection Primer Set. Human (manufactured by TAKARA BIO INC.). The results of the measurement are shown in Fig. 6. In Fig. 6, the ordinate axis indicates the copy number of the transferred gene in a gene-transferred cell. As a result, the copy number was increased by a combination of stimulation by an anti-CD3 antibody alone or fibronectin fragment/anti-CD3 antibody stimulation with addition of retinoic acid, and the increase of the copy number correlated with increase in gene transfer efficiency.

Example 3 Preparation of ZsGreen gene-transferred cell

(1) Preparation of virus liquid

A retrovirus vector plasmid pDON-AI-ZsGreen was prepared by the following procedure. First, a ZsGreen expression vector pZsGreen-N1 (manufactured by Clontech) was digested with the restriction enzyme NotI, blunted using DNA Blunting Kit (manufactured by TAKARA BIO INC.) and then digested with the restriction enzyme BamHI to obtain an about 720 bp fragment of the ZsGreen gene. Then, the ZsGreen DNA fragment was inserted into a vector obtained by digesting a retrovirus vector plasmid pDON-AI (manufactured by TAKARA BIO INC.) with the restriction enzymes BamHI and HpaI, using DNA Ligation Kit Mighty Mix (manufactured by TAKARA BIO INC.) to obtain a ZsGreen expression recombinant retrovirus vector pDON-AI-ZsGreen.

Transient virus preparation using the pDON-AI-ZsGreen vector and Retrovirus Packaging Kit Eco (manufactured by TAKARA BIO INC.) was carried out to obtain an ecotropic DON-AI-ZsGreen virus. A GALV retrovirus packaging cell PG13 (ATCC CRL-10686) was infected with The ecotropic DON-AI-ZsGreen virus in the presence of polybrene to obtain a gene-transferred cell PG13/DON-AI-ZsGreen. The PG13/DON-AI-ZsGreen was cultured in a Dulbecco's modified Eagle medium (DMEM, manufactured by Sigma) containing 10% bovine fetal serum (manufactured by Invitrogen), and grown to semiconfluent. At that time, the medium was exchanged with 0.1 mL/cm$^2$ of a fresh 10% bovine fetal serum-containing DMEM. After 24 hours, the supernatant was filtered with a 0.45 $\mu$m filter (manufactured by Millipore) to obtain a GALV/DON-AI-ZsGreen virus liquid. The resulting virus liquid was aliquoted, dispensed, and stored in a -80°C freezer until just before use for preparation of the following gene-transferred cell. The titer of the virus liquid was $6.76 \times 10^5$ cfu/mL.

(2) Preparation of ZsGreen gene-transferred cell

In the same ways as in Example 1, a fibronectin fragment/anti-CD3 antibody-adherent plate and an anti-CD3 antibody-adherent plate were prepared.

A PBMC prepared from a healthy person from whom informed consent had been obtained was suspended at $0.2 \times 10^6$ cells/mL in a GT-T503 medium containing 1% autologous plasma, 720 IU/mL IL-2, 0.2% HSA, 2.5 $\mu$g/mL Fungizone and 10 nM retinoic acid, and added to the fibronectin fragment/anti-CD3 antibody-adherent plate or the anti-CD3 antibody-adherent plate at 2.6 mL/well. As controls, a cell suspension not containing a retinoic acid was added to the plates. Each plate was cultured at 37°C for 4 days in a $CO_2$ incubator.

A virus-bound plate for gene transferred was prepared as follows.
A fibronectin fragment was dissolved in PBS at 20 $\mu$g/mL. This solution was added to a tissue culture-untreated 24-well plate in an amount of 0.5 mL/well, and allowed to stand at 4°C overnight. Then, the solution was removed, and the plate was washed twice using 1 mL/well of PBS. To the wells, 1 mL of the GALV/DON-AI-ZsGreen virus liquid prepared in the above (1) was added, and then centrifuged at 32°C and $2000 \times$ g for 2 hours. After centrifugation, the virus liquid was removed, and the wells were washed three times using 2 mL/well of 1.5% HSA-containing PBS to prepare a virus-bound plate.

A gene transfer operation was carried out as follows. The PBMC which had been stimulated with an anti-CD3 antibody alone or a fibronectin fragment/an anti-CD3 antibody for 4 days was added to the virus-bound plate at $0.8 \times 10^6$ cells/0.80 mL/well as suspension in a GT-T-RetroIII medium containing 5 mM sodium butyrate. The plate was cultured at 37°C for 4 hours in a $CO_2$ incubator. The cells after culturing were suspended at $0.1 \times 10^6$ cells/mL in a GT-T503 medium containing 1% autologous plasma, 720 IU/mL IL-2, 0.2% HSA, 2.5 $\mu$g/mL Fungizone and 10 nM retinoic acid. The resulting suspension was seeded on a 12-well plate, and cultured at 37°C for 3 days. At the same time, a gene transfer operation and cell culture after gene transfer were similarly carried out using a cell suspension not containing retinoic acid as a control. Further, gene transfer by a polybrene method without a virus-bound plate for gene transfer was carried out using the ZsGreen virus liquid. The PBMC which had been stimulated for 4 days was suspended at $1.0 \times 10^6$ cells/mL in the GALV/DON-AI-ZsGreen virus liquid prepared in the above (1). To this suspension was added polybrene (Hexadimethrine Bromide, manufactured by SIGMA-ALDRICH) at the final concentration of 8 $\mu$g/mL. This suspension was added to a 24-well plate in an amount of 2 mL/well, and centrifuged at 32°C and $1000 \times$ g for 1 hour. After centrifugation, the virus liquid was removed, and seeded on a tissue culture-treated 12-well plate at $0.53 \times 10^6$ cells/2.65 mL/well, and then cultured at 37°C for 2 days.

Regarding gene transfer using a virus-bound plate for gene transfer, the cell growth rate was measured on the 4th and 7th days, and cell surface markers, the proportion of ZsGreen positive cells and the copy number of a transferred gene were measured on the 7th day. Regarding the cells into which the gene was transferred by a polybrene method, the proportion of ZsGreen positive cells was measured on the 6th day.

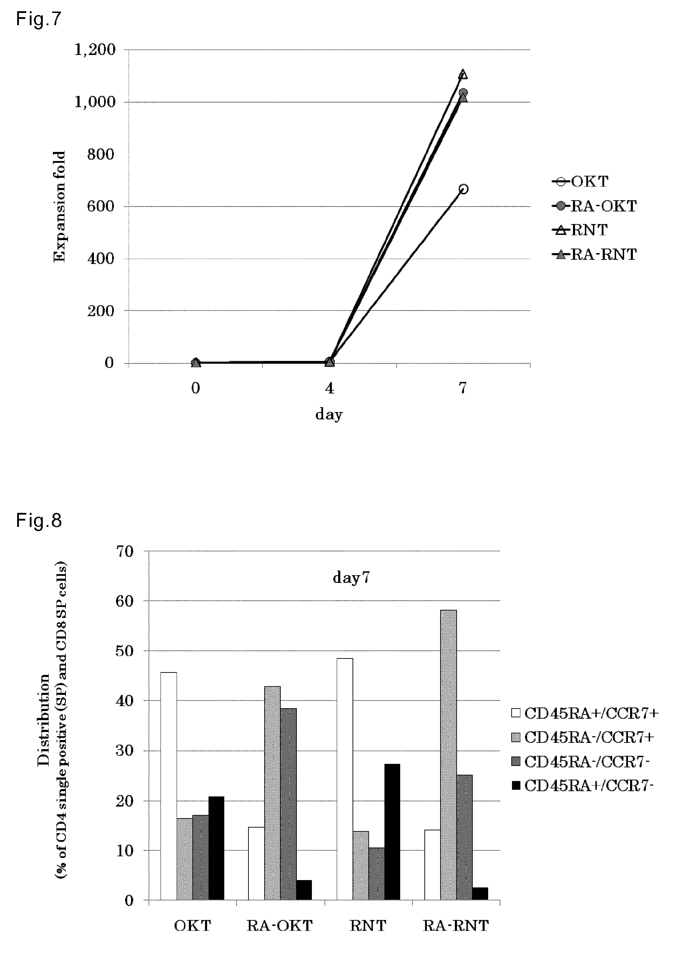

The results of measurement of the cell growth rate are shown in Fig. 7. The ordinate axis in Fig. 7 indicates the growth ratio (%) relative to the cell number at initiation of culture, and the abscissa axis indicates the number of days of culture. As seen from Fig. 7, the cell growth rate was increased by stimulation by an anti-CD3 antibody alone or fibronectin fragment/anti-CD3 antibody stimulation, regardless of the presence or absence of retinoic acid.

The results of measurement of cell surface markers in CD8 single positive and CD4 single positive cells in the cultured cells using an anti-CD4 antibody, an anti-CD8 antibody, an anti-CD45RA antibody and an anti-CCR7 antibody are shown in Fig. 8. Under each stimulation condition of Fig. 8, bars represent the proportions (%) of CD45RA+/CCR7+, CD45RA-/CCR7+, CD45RA-/CCR7- and CD45RA+/CCR7- cells in CD8 single positive and CD4 single positive cells from left. As seen from Fig. 8, the proportion of central memory T-like cells and the proportion of effector memory T-like cells were increased by a combination stimulation by an anti-CD3 antibody alone or fibronectin fragment/anti-CD3 antibody stimulation with retinoic acid stimulation.

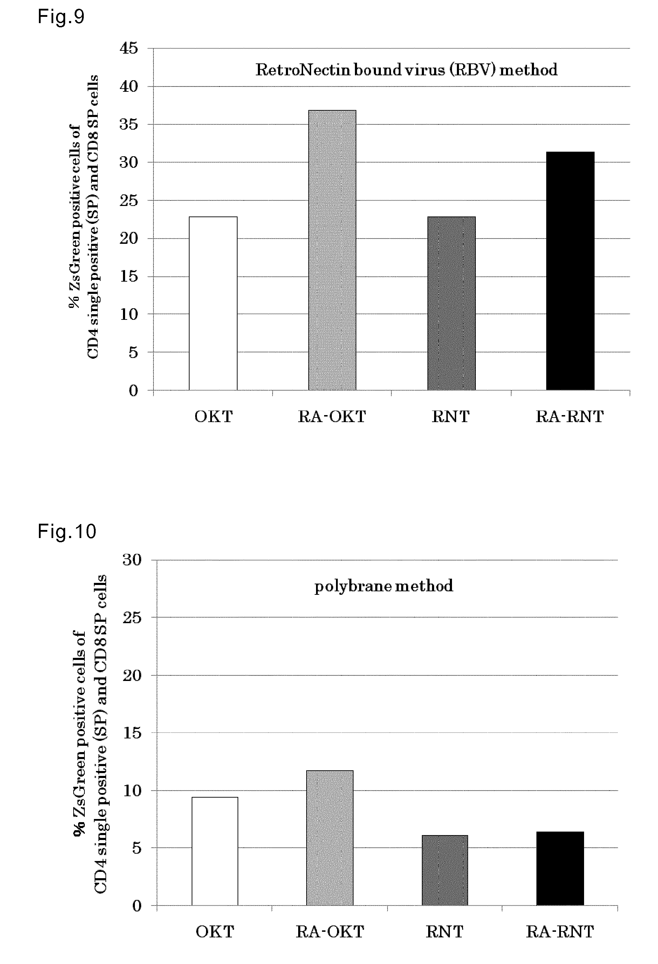

The proportion of ZsGreen positive cells was measured.
After initiation of stimulation of the PBMC, the cells on the 7th day in the case of gene transfer using the virus-bound plate for gene transfer, and the cells on the 6th day in the case of gene transfer by a polybrene method were reacted with a PE-Cy7-labeled anti-human CD4 antibody or an APC-Cy7-labeled anti-human CD8 antibody (manufactured by Becton Dickinson) at 4°C for 20 minutes, washed and, then subjected to flow cytometry analysis using FACScant (manufactured by Becton Dickinson). The measurement results are shown in Fig. 9 and Fig. 10. The ordinate axis in Fig. 9 and Fig. 10 indicates the proportion (%) of ZsGreen positive cells in CD8 single positive and CD4 single positive cells. As a result, in the case of gene transfer using the virus-bound plate for gene transfer shown in Fig. 9, a combination of stimulation by an anti-CD3 antibody alone or fibronectin fragment/anti-CD3 antibody stimulation with addition of retinoic acid increased the proportion of ZsGreen positive cells by 1.5-fold as compared with the no addition of retinoic acid. On the other hand, in the case of gene transfer by a polybrene method shown in Fig. 10, there was little difference in transfer efficiency, regardless of the presence or absence of retinoic acid stimulation in combination with stimulation by an anti-CD3 antibody alone or fibronectin fragment/anti-CD3 antibody stimulation. As seen from Fig. 9 and Fig. 10, gene transfer with a high efficiency was shown by a combination of retinoic acid stimulation and a gene transfer method using a fibronectin fragment.

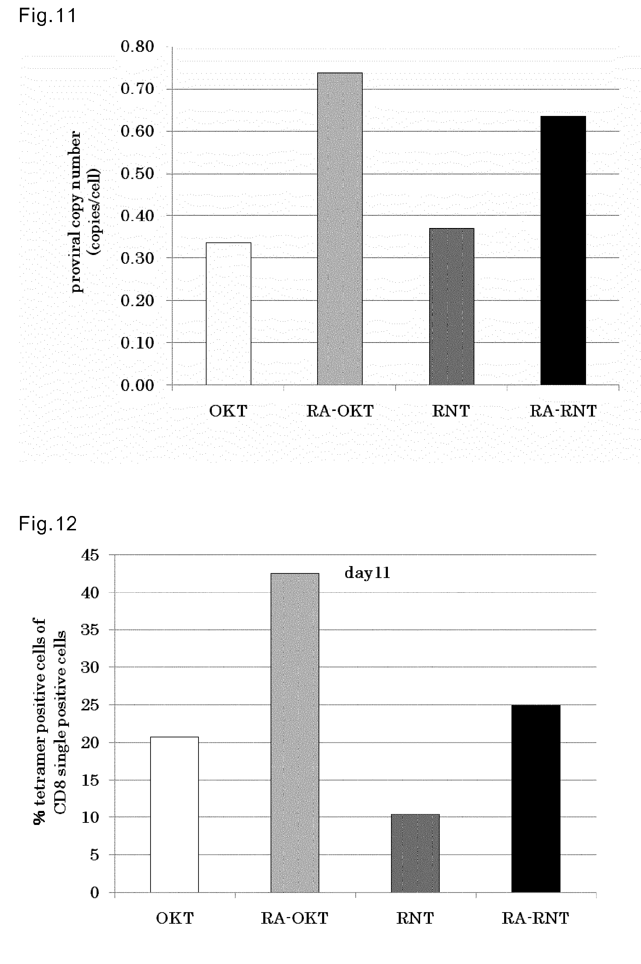

The results of measurement of the copy number of the transferred gene in the gene-transferred cell into which the gene was transferred using the virus-bound plate for gene transfer are shown in Fig. 11. In Fig. 11, the ordinate axis indicates the copy number of the transferred gene in the gene-transferred cell. As a result, the copy number under the combination condition of stimulation by an anti-CD3 antibody alone or fibronectin fragment/anti-CD3 antibody stimulation with addition of retinoic acid was about 2 times the copy number under the condition of no addition of retinoic acid. The copy number correlated with gene transfer efficiency.

Example 4 Measurement of anti-tumor activity of TCR gene-transferred cell

In the same ways as in Example 1-(1) to (4) of WO2008/11150 a series of operations of cloning of a MAGE-A4-specific TCR gene, construction of a vector and preparation of a producer cell were carried out to prepare a MS-bPa virus liquid. The RNA copy number of the present virus liquid was $2.55 \times 10^{11}$ copies/mL.

(1) Preparation of TCR gene-transferred cell

In the same ways as in Example 2-(1), a fibronectin fragment/anti-CD3 antibody-adherent plate and an anti-CD3 antibody-adherent plate were prepared.

A PBMC of a healthy person from whom informed consent had been obtained was suspended at $0.20 \times 10^6$ cells/mL in a GT-T503 medium containing 1% autologous plasma, 720 IU/mL IL-2, 0.2% HSA, 2.5 $\mu$g/mL Fungizone and 10 nM retinoic acid. Then, 2.65 mL of the suspension was added to the fibronectin fragment/anti-CD3 antibody-adherent plate or the anti-CD3 antibody-adherent plate. As controls, a cell suspension not containing retinoic acid was used to prepare plates. Each plate was cultured at 37°C for 4 days in a $CO_2$ incubator.

A virus-bound plate for gene transfer was prepared as follows. A fibronectin fragment was dissolved in PBS at 20 $\mu$g/mL. This solution was added to a tissue culture-untreated 24-well plate at 0.5 mL/well, and allowed to stand at 4°C for 4 days. Then, the solution was removed, and the plate was washed twice using 1 mL/well of PBS. To the wells, 0.9 mL of the MS-bPa virus liquid was added and centrifuged at 32°C and $2000 \times$ g for 2 hours. After centrifugation, the virus liquid was removed, and the plate was washed three times using 1 mL/well of 1.5% HSA-containing PBS to prepare a virus-bound plate.

A gene transfer operation was carried out as follows. The PBMC which had been stimulated with an anti-CD3 antibody alone or a fibronectin fragment/an anti-CD3 antibody for 4 days was added to the virus-bound plate at $0.8 \times 10^6$ cells/2.0 mL/well as suspension in a GT-T-RetroIII medium containing 5 mM sodium butyrate, and cultured at 37°C overnight in a $CO_2$ incubator. The cells after culturing were suspended at $0.8 \times 10^6$ cells/1.0 mL/well in a GT-T503 medium containing 1% autologous plasma, 720 IU/mL IL-2, 0.2% HSA, 2.5 $\mu$g/mL Fungizone and 10 nM retinoic acid, seeded on a fresh virus-bound plate, and cultured at 37°C for 4 hours in a $CO_2$ incubator. After completion of culture, the cells were recovered, seeded again on a 12-well plate for cell culture at $0.1 \times 10^6$ cells/mL, and cultured at 37°C for 3 days in a $CO_2$ incubator. At the same time, a gene transfer operation and cell culture after gene transfer were similarly carried out using a cell suspension not containing retinoic acid as a control. On the 8th day after initiation of stimulation of the PBMC, the cells were seeded on a 12-well plate for cell culture at $0.1 \times 10^6$ cells/mL, and cultured until the 11th day. On the 10th day, analysis of cytotoxicity on the transferred TCR-specific peptide was carried out. On the 11th day, expression of the transferred TCR in CD8 single positive cells, and the proportion of IL-4 and perforin-producing cells were measured.

In the same ways as in Example 2-(1), expression of the MAGE-A4-specific TCR was measured by a tetramer assay.

The results of tetramer measurement are shown in Fig. 12. In Fig. 12, the ordinate axis is the tetramer positive proportion (%). As a result, the expression of the MAGE-A4-specific TCR in both the group of stimulation by an anti-CD3 antibody alone and the group of fibronectin fragment/anti-CD3 antibody stimulation was increased by retinoic acid stimulation, that is, gene transfer efficiency was increased by retinoic acid stimulation.

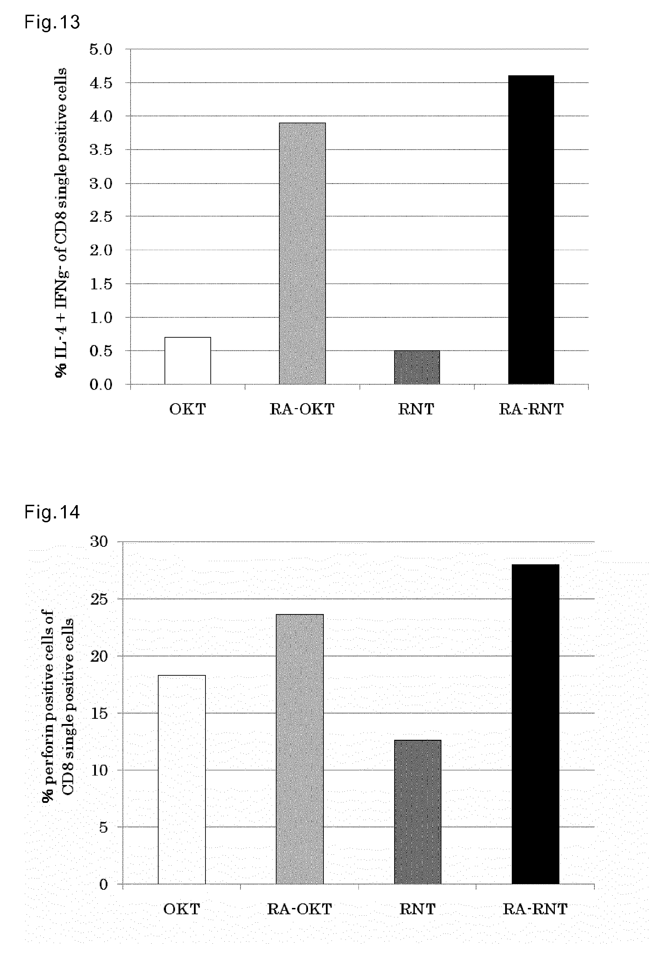

The proportion of IL-4 and perforin-producing cells in the gene-transferred cells in response to a MAGE-A4-derived peptide p143 was measured. The cells on the 11th day and MAGE-A4-derived peptide-bound T2A24 cells [Clinical Cancer Research, vol. 11, pp. 5581-5589 (2005)] were co-cultured at the ratio of 1 : 1 for 2 hours. After Brefeldin A (manufactured by Sigma Aldrich) was added, the co-culture was further continued for 2 hours, and the reaction was stopped with a 10-fold amount of cold PBS. These cells was stained with an APC-Cy7-labeled anti-human CD8 antibody (manufactured by Becton Dickinson) at 4°C for 20 minutes. Then, after the cells were fixed and permeated using IntraPrep (manufactured by Beckman Coulter), the cells were stained with a PE-Cy7-labeled anti-human IFN-γ antibody (manufactured by Becton Dickinson), an APC-labeled anti-human perforin antibody (manufactured by Becton Dickinson) and a PE-labeled anti-human IL-4 antibody (manufactured by Beckman Coulter) at room temperature for 20 minutes. After the cells were washed, flow cytometry analysis was carried out using FACScant (manufactured by Becton Dickinson). The results of the proportion of IL-4-producing and IFN-γ-nonproducing cells are shown in Fig. 13. The results of the proportion of perforin-producing cells are shown in Fig. 14. That is, the ordinate axis in Fig. 13 indicates the proportion (%) of IL-4-producing and IFN-γ-nonproducing cells, and the ordinate axis in Fig. 14 indicates the proportion (%) of perforin-producing cells.

In addition, in each graph of Figs. 13, 19, 27 and 28, IFN-γ is described as IFNg.

As a result, the proportion of IL-4-producing and IFN-γ-nonproducing cells and the proportion of perforin-producing cells were increased by retinoic acid stimulation, in both the group of stimulation by an anti-CD3 antibody alone and the group of fibronectin fragment/anti-CD3 antibody stimulation. From these results, the memory T-like cell of the present invention is classified into a Tc2-type cell.

Analysis of cytotoxicity of the gene-transferred cell on a MAGE-A4-derived peptide p143 was carried out. After the T2A24 cells which had been co-cultured with the peptide for 3 hours was suspended at $0.5 \times 10^6$ cells/mL in a RPMI 1640 medium containing 10% FBS (fetal bovine serum, manufactured by SAFC), Calcein-AM [manufactured by Dotite] was added at the final concentration of 25 μM. The suspension was cultured at 37°C for 1 hour. The cells were washed with a medium not containing Calcein-AM, and mixed with a 10-fold amount of K562 cells (ATCC CCL-243) to prepare Calcein-labeled target cells. The K562 cells were used in order to exclude nonspecific cytotoxic activity due to NK cells commingling with responder cells. The cells on the 10th day after initiation of culture as effector cells were stepwisely diluted with a RPMI 1640 medium containing 10% FBS so as to be $1 \times 10^3$ to $1 \times 10^6$ cells/mL. Then, 100 μL/well of the dilution was dispensed into each well of a 96-well cell culture plate. The Calcein-labeled target cells were adjusted to $1 \times 10^5$/mL and added to the wells at 100 μL/well. The plate containing the cell suspension was centrifuged at $180 \times g$ for 2 minutes, and incubated at 37°C for 4 hours in a $CO_2$ incubator. After 4 hours, the plate containing the cell suspension was centrifuged at $180 \times g$ for 2 minutes. From the each well, 100 μL of the culture supernatant was removed, and the amount of Calcein released into the culture supernatant was measured with a fluorescent plate reader (485 nm/538 nm). "Specific cytotoxic activity (%)" was calculated according to the following mathematical formula 1.

```
[Mathematical formula 1]

Specific cytotoxic activity (%) =

[{(measured value of each well) - (minimum released

amount)}/{(maximum released amount) - (minimum released


amount)}] × 100
```

In the above-mentioned equation, the minimum released amount is the released amount of Calcein in wells containing only the target cells and K562 cells, and indicates the amount of Calcein naturally released from the target cells. The maximum released amount indicates the released amount of Calcein when 0.1% surfactant Triton X-100 (manufactured by Nacalai Tesque) is added to the target cells to completely destroy the cells.

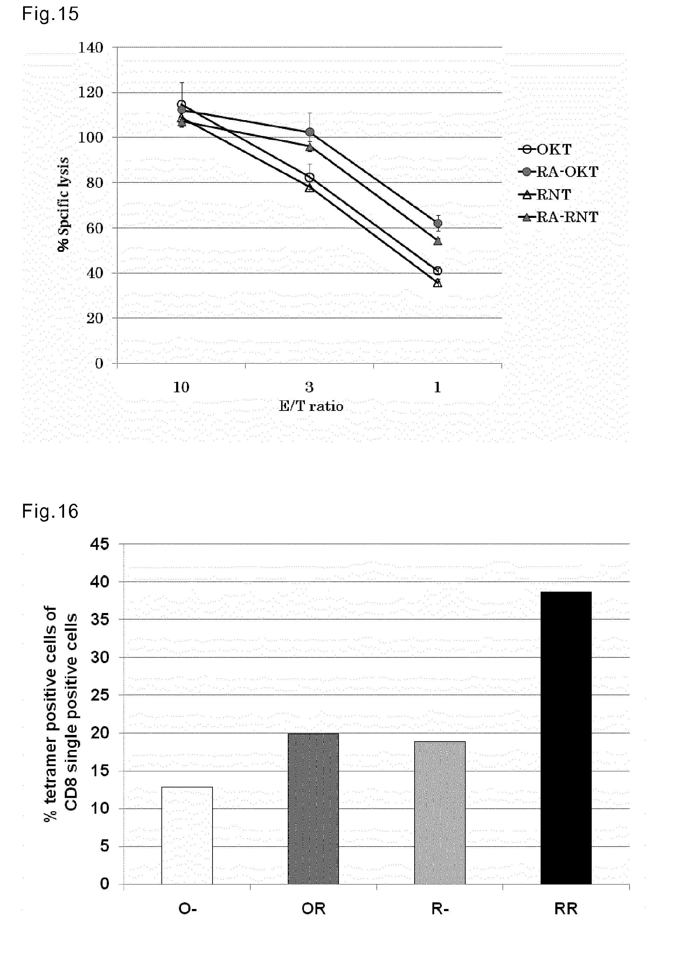

The results of analysis of cytotoxicity of the gene-transferred cell on a MAGE-A4-derived peptide p143 are shown in Fig. 15. The abscissa axis in Fig. 15 indicates the ratio of the PBMC recovered on the 10th day after initiation of culture to the T2A24 cell pulsed with MAGE-A4 (E/T ratio). As a result, when cytotoxic activities (%) in the presence or absence of retinoic acid stimulation in combination with stimulation by an anti-CD3 antibody alone or fibronectin fragment/anti-CD3 antibody stimulation were compared, the cytotoxic activity on the specific target cells in the retinoic acid stimulation group was high. It was found that anti-tumor activity was increased in the retinoic acid stimulation group.

Example 5 Effect of timing of adding retinoic acid on property of TCR gene-transferred cell

In the same ways as in Example 1-(1) to (4) of WO2008/11150 a series of operations of cloning of a MAGE-A4-specific TCR gene, construction of a vector and preparation of a producer cell were carried out to prepare a MS-bPa virus liquid. The RNA copy number of the present virus liquid was $2.55 \times 10^{11}$ copies/mL.

(1) Preparation of TCR gene-transferred cell

In the same ways as in Example 2-(1), an anti-CD3 antibody-adherent plate was prepared.

A PBMC of a healthy person from whom informed consent has been obtained was suspended at $0.20 \times 10^6$ cells/mL in a GT-T503 medium containing 1% autologous plasma, 720 IU/mL IL-2, 0.2% HSA, 2.5 $\mu$g/mL Fungizone and 10 nM retinoic acid. Then 2.65 mL of the resulting suspension was added to the anti-CD3 antibody-adherent plate. As a control, a cell suspension not containing retinoic acid was used to prepare a plate. Each plate was cultured at 37°C for 4 days in a $CO_2$ incubator.

A virus-bound plate for gene transfer was prepared as follows.
A fibronectin fragment was dissolved in PBS at 20$\mu$ g/mL. This solution was added to a tissue culture-untreated 12-well plate in an amount of 1 mL/well, and allowed to stand at 4°C for 4 days. Then, the solution was removed, and the plate was washed twice using 2 mL/well of PBS. Then, 1.8 mL of the MS-bPa virus liquid was added to the wells, and the plate was centrifuged at 32°C and 2000 $\times$ g for 1.5 hours. After centrifugation, the virus liquid was removed, and the plate was washed three times using 1 mL/well of 1.5% HSA-containing PBS to prepare a virus-bound plate.

A gene transfer operation was carried out as follows. The PBMC which had been stimulated with an anti-CD3 antibody for 4 days was added to the virus-bound plate at $1.6 \times 10^6$ cells/4.0 mL/well as suspension in a GT-T503 medium containing 1% autologous plasma, 720 IU/mL IL-2, 0.2% HSA, 2.5 $\mu$g/mL Fungizone and 10 nM retinoic acid, and cultured at 37°C overnight in a $CO_2$ incubator. At the same time, a cell suspension not containing retinoic acid of the PBMC which had been stimulated with an anti-CD3 antibody for 4 days was used to prepare a plate. Using the control plate, a gene transfer operation and cell culture after gene transfer were similarly carried out.

The gene-transferred cells after culturing overnight were recovered, and suspended at $0.1 \times 10^6$ cells/mL in a GT-T503 medium containing 1% autologous plasma, 720 IU/mL IL-2, 0.2% HSA and 2.5 $\mu$g/mL Fungizone which contained retinoic acid at the final concentration of 10 nM or did not contain. The resulting suspension was seeded on a 12-well plate for cell culture in an amount of 4.0 mL/well, and cultured at 37°C for 3 days in a $CO_2$ incubator.
On the 8th day after initiation of stimulation of the PBMC, the cells were recovered and, subsequently, suspended at $0.1 \times 10^6$ cells/mL in a GT-T503 medium containing 1% autologous plasma, 720 IU/mL IL-2, 0.2% HSA and 2.5 $\mu$g/mL Fungizone which contained retinoic acid at the final concentration of 10 nM or did not contain. The suspension was seeded on a 12-well plate for cell culture, and cultured until the 11th day. On the 11th day, measurement of the proportion of cells expressing cell surface markers and the transferred TCR in CD8 single positive cells, and the proportion of IL-4-, IFN-$\gamma$- and perforin-producing cells in response to the transferred TCR-specific peptide, and analysis of cytotoxicity were carried out.

In each graph of Figs. 16 to 25, "RR" indicates the group of cells cultured with addition of retinoic acid from initiation of stimulation of PBMC to the 11th day, "R-" indicates the group of cells cultured with addition of retinoic acid from initiation of stimulation of PBMC until gene transfer (5th day) and cultured without adding retinoic acid after gene transfer, "OR" indicates the group of cells cultured without adding retinoic acid from initiation of stimulation of PBMC until gene transfer (5th day) and cultured with addition of retinoic acid after gene transfer, and "O-" indicates the group of cells cultured without adding retinoic acid from initiation of stimulation of PBMC until the 11th day.

In the same ways as in Example 2-(1), expression of the MAGE-A4-specific TCR was measured by a tetramer assay.

The results of tetramer measurement are shown in Fig. 16. In Fig. 16, the ordinate axis indicates the tetramer positive proportion (%) in CD8 single positive cells. As a result, the tetramer positive cell proportion was the highest in "RR" in which retinoic acid was added during cell culture, the second highest in "OR" in which retinoic acid was added after gene transfer, the third highest in "R-" in which the cells were cultured with addition of retinoic acid until gene transfer, and the lowest in "O-" in which the cells were cultured without adding retinoic acid. Since "R-" had a higher positive proportion than "O-", it was made clear that gene transfer efficiency was increased by addition of retinoic acid. Since "OR" had a higher positive proportion than "O-" and "RR" had a higher positive proportion than "R-", it was made clear that expression of a transferred gene was also increased by addition of retinoic acid.

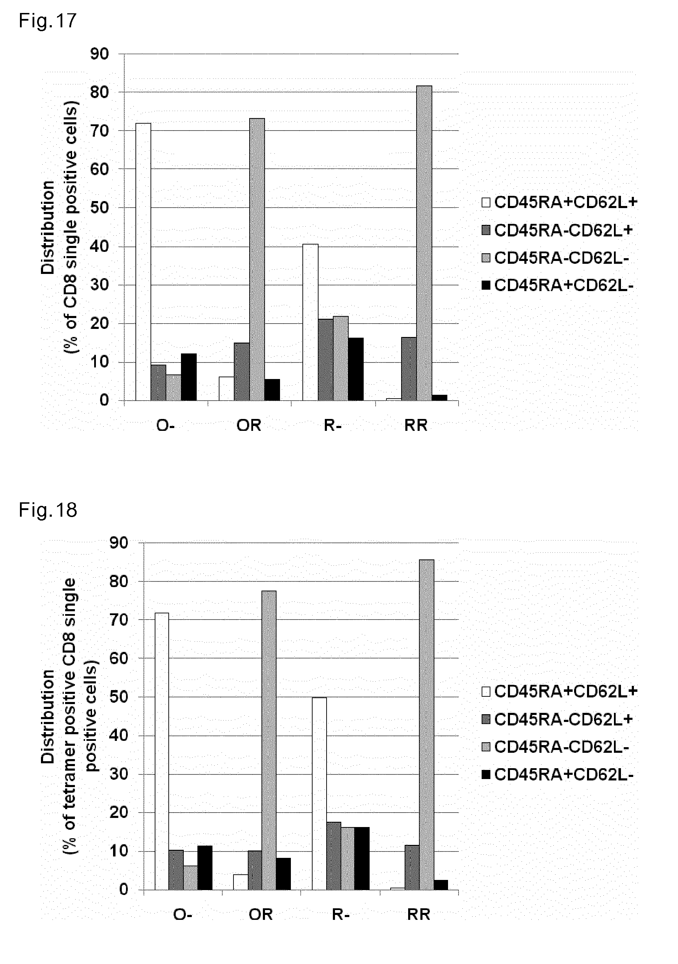

The results of measurement of cell surface markers of CD8 single positive cells are shown in Fig. 17, and the results of measurement of cell surface markers of CD8 single positive and tetramer positive cells are shown in Fig. 18. The proportion of memory T cells was measured by staining the cells with an anti-CD8 antibody, an anti-CD45RA antibody and an anti-CD62L antibody. In each cell group of Fig. 17 and Fig. 18, bars represent the proportions (%) of CD45RA+/CD62L+ (naive T-like cell), CD45RA-/CD62L+ (central memory T-like cell), CD45RA-/CD62L-(effector memory T-like cell) and CD45RA+/CD62L- (effector T-like cell) from left. As seen from Fig. 17 and Fig. 18, when retinoic acid was added, the proportions of central memory T-like cells and effector memory T-like cells were increased as compared with the case of no addition (O-) .

In the same ways as in Example 4-(1), the proportion of IL-4 and perforin-producing cells in the gene-transferred cells in response to a MAGE-A4-derived peptide p143 was measured.

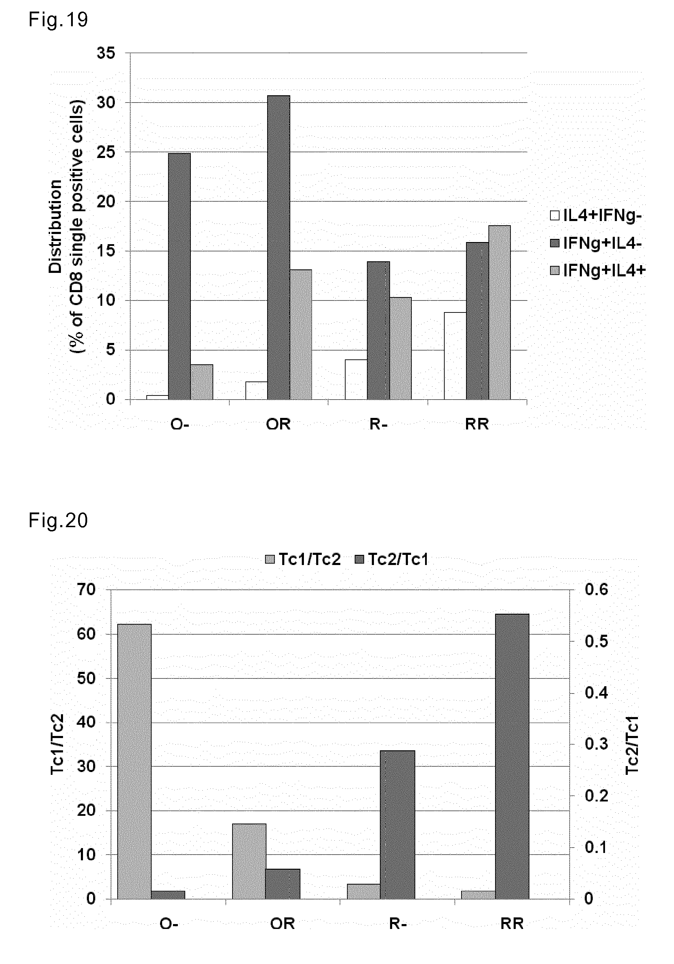

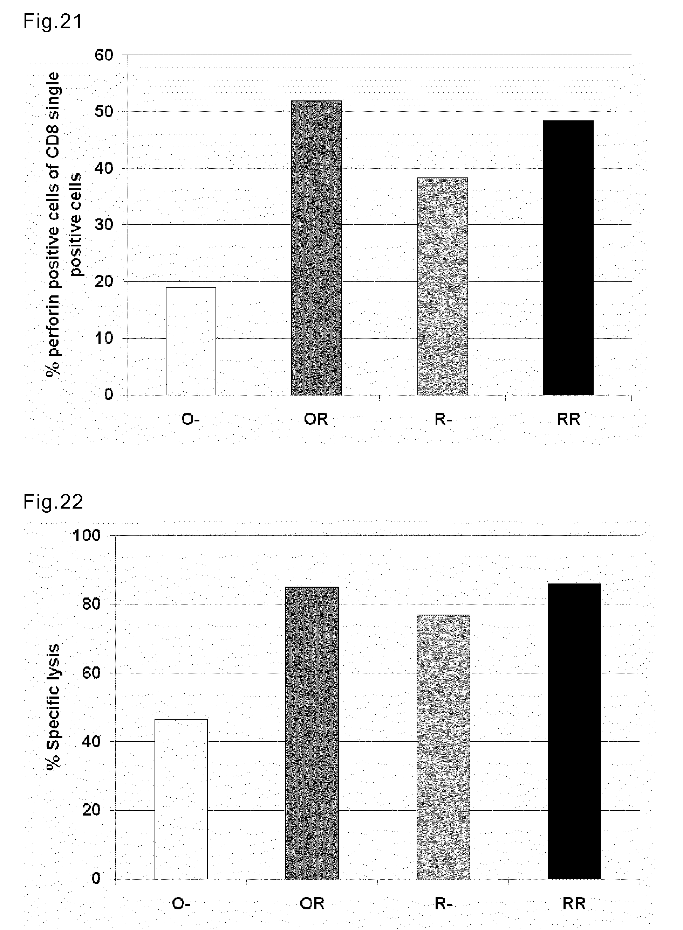

The results of the proportion of IL-4-producing or IL-4-nonproducing and IFN-γ-producing or IFN-γ-nonproducing cells in CD8 single positive cells are shown in Fig. 19. The ratio between the proportion of IL-4-producing and IFN-γ-nonproducing cells (Tc2) and the proportion of IL-4-nonproducing and IFN-γ-producing cells (Tc1) is shown in Fig. 20. The results of the proportion of perforin-producing cells in CD8 single positive cells are shown in Fig. 21. In each cell group of Fig. 19, bars represent the proportions (%) of IL-4-producing and IFN-γ-nonproducing cells, IL-4-nonproducing and IFN-γ-producing cells, and IL-4-producing and IFN-γ-producing cells from left. In each cell group of Fig. 20, bars represent Tc1/Tc2 and Tc2/Tcl from left, and in the scale of the ordinate axis, the left side (0 to 70) corresponds to Tc1/Tc2 and the right side (0 to 0.6) corresponds to Tc2/Tc1.

As a result, as seen from Fig. 19, "RR" showed the highest proportion of cells producing IL-4 which is a type II cytokine, and "OR" showed the highest proportion of cells producing IFN-γ which is a type I cytokine. As compared with "O-" representing no addition, "R-", "RR" and "OR" showed high proportions of IL-4-producing and IFN-γ-producing cells. As seen from Fig. 20, since the ratio of Tc2 and Tc1 was higher in "R-" than in "OR", it was made clear that the presence of retinoic acid during culture before gene transfer was important for inducing a Tc2-type CTL. In addition, the proportion of perforin-producing cells was high in the order of "OR", "RR", "R-" and "O-". From the above-mentioned results, it was made clear that the memory T-like cell prepared by the present invention can be led to a Tc1-type or Tc2-type CTL by controlling the timing of addition of retinoic acid, and a cell population exhibiting a higher proportion of perforin-producing cells could be prepared by adding retinoic acid.

According to the same manner as that of Example 4-(1) except that cells on the 11th day after initiation of culture were stepwisely diluted with a RPMI 1640 medium containing 10% FBS to $1 \times 10^5$ cells/mL as an effector cell and then 100 μL/well of the dilution was dispensed into each well of a 96-well cell culture plate, cytotoxicity of the gene-transferred cell on a MAGE-A4-derived peptide p143 was analyzed.

The results of analysis of cytotoxicity of the gene-transferred cell on a MAGE-A4-derived peptide p143 are shown in Fig. 22. Fig. 22 shows a cytotoxicity rate when the PBMC recovered on the 11th day after initiation of culture and the T2A24 cell pulsed with MAGE-A4 were co-cultured at the ratio of 1 : 1. As a result, when cytotoxic activities (%) in the presence or absence of addition of retinoic acid were compared, the cytotoxic activity on the specific target cells in the cell groups with retinoic acid stimulation ("RR", "OR", and "R-") was higher than that in the cell group without retinoic acid stimulation ("O-"), and it was found that anti-tumor activity was increased by retinoic acid stimulation.

Example 6 Gene transfer efficiency and transferred gene expressing effect due to addition of retinoic acid

In the same ways as in Example 1-(1) to (4) of WO2008/111506, a series of operations of cloning of a MAGE-A4-specific TCR gene, construction of a vector and preparation of a producer cell were carried out to prepare a MS-bPa virus liquid. The RNA copy number of the present virus liquid was $2.55 \times 10^{11}$ copies/mL.

(1) Preparation of TCR gene-transferred cell

In the same ways as in Example 5-(1), a series of operations of preparation of an anti-CD3 antibody-adherent plate, culture of a PBMC before gene transfer, preparation of a virus-bound plate for gene transfer, an operation of transferring a TCR gene into a PBMC which had been stimulated with an anti-CD3 antibody for 4 days, and cell culture after TCR gene transfer were carried out to prepare a TCR gene-transferred cell.

On the 11th day from initiation of stimulation of the PBMC, the proportion of cells expressing the transferred TCR, the expression amount and the provirus copy number were measured.

In the same ways as in Example 2-(1), expression of the MAGE-A4-specific TCR was measured by a tetramer assay.

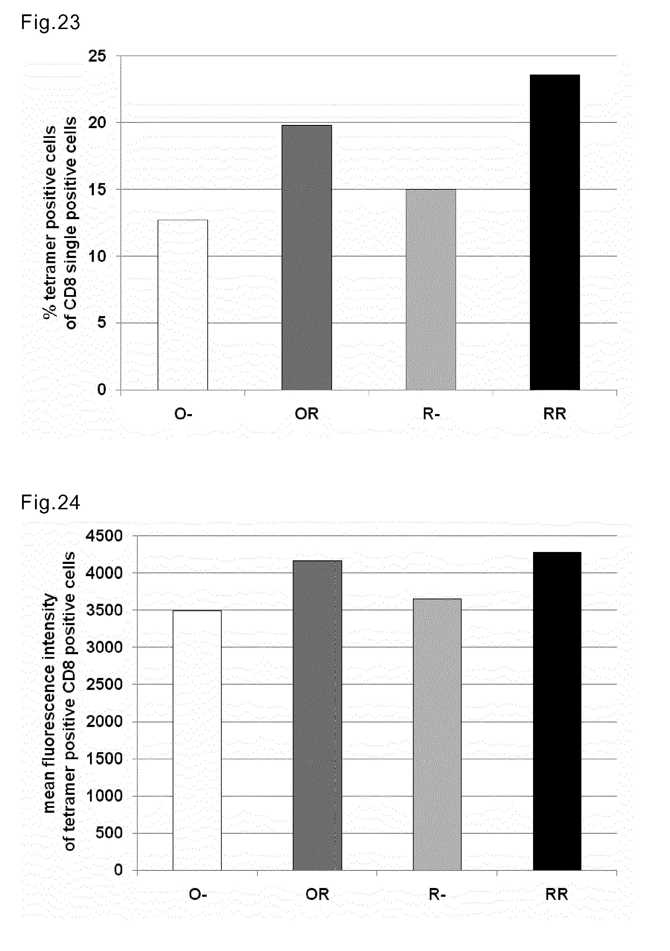

The results of tetramer measurement are shown in Fig. 23 and Fig. 24. In Fig. 23, the ordinate axis indicates the tetramer positive proportion (%). In Fig. 24, the ordinate axis indicates the expression amount of a tetramer in tetramer positive and CD8 single positive cells as an average fluorescent intensity value. As a result, the proportion of tetramer positive cells was the highest in "RR" in which the cells were cultures with addition of retinoic acid from the initiation to the end, the second highest in "OR" in which retinoic acid was added after gene transfer, the third highest in "R-" in which the cells were cultured with addition of retinoic acid until gene transfer and cultured without addition of retinoic acid after gene transfer, and the lowest in "O-" in which cells were cultured without addition of retinoic acid. In addition, the tetramer expression amount, that is, the average fluorescent intensity in CD8 single positive and tetramer positive cells showed a result correlated with the result of the tetramer positive cell proportion. That is, cells having an increased positive cell proportion and an increased expression amount of the transferred cell could be obtained by adding retinoic acid in the preparation process particularly in the cell groups in which retinoic acid was added, after gene transfer ("OR" and "RR"), as compared with the cell group in which retinoic acid was not added ("O-").

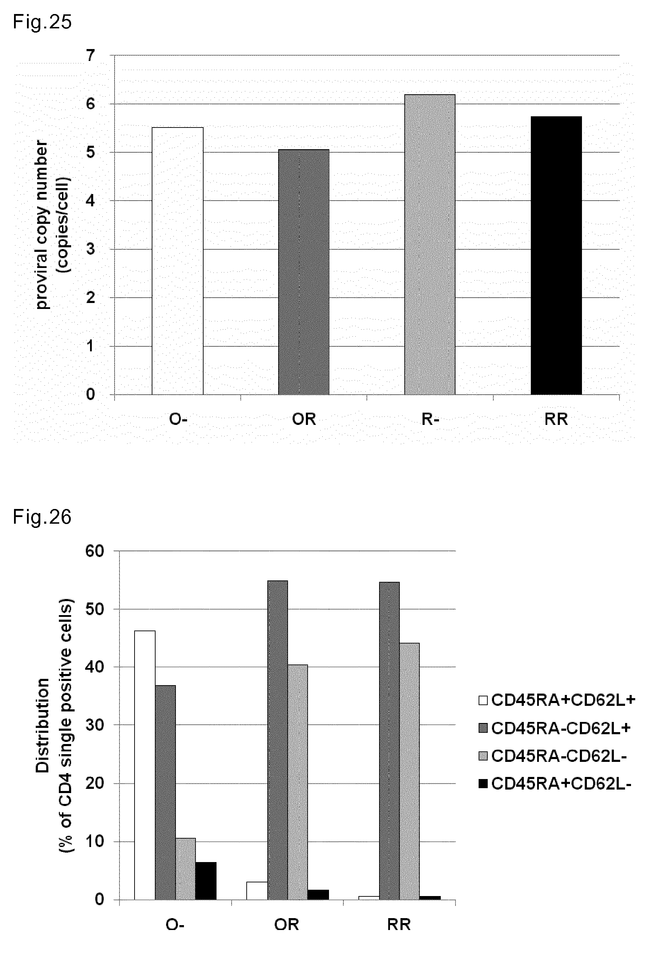

The provirus copy number of the transferred gene in the cultured cells was measured using Provirus Copy Number Detection Primer Set. Human (manufactured by TAKARA BIO INC.). The results of the measurement are shown in Fig. 25. The ordinate axis in Fig. 25 indicates the copy number of the transferred gene in the cultured cells. As a result, the provirus copy number was increased in "R-" in which gene transfer was carried out in the presence of retinoic acid, as compared with "O-" in which gene transfer was carried out without addition of retinoic acid. That is, it was made clear that gene transfer efficiency was increased by adding retinoic acid before gene transfer.

On the other hand, there was slight difference in the provirus copy number between the cell groups in which the cells were cultured in the same well until gene transfer (5th day) ("O-" and "OR" or "R-" and "RR"). In contrast, as seen from the results of Fig. 23 and Fig. 24, the proportion of tetramer positive cells and the expression amount thereof were higher in the cell groups in which retinoic acid was added after gene transfer ("OR" and "RR") than in the cell groups in which retinoic acid was not added ("O-" and "R-"). That is, it was made clear that expression of a transferred gene was increased by adding retinoic acid after gene transfer.

Example 7 Effect of retinoic acid addition on properties of CD4 single positive cells

In the same ways as in Example 2-(1), an anti-CD3 antibody-adherent plate was prepared.

A PBMC of a healthy person from whom informed consent had been obtained was suspended at $0.20 \times 10^6$ cells/mL in a GT-T503 medium containing 1% autologous plasma, 720 IU/mL IL-2, 0.2% HSA, 2.5 $\mu$g/mL Fungizone and 10 nM retinoic acid. The suspension was added to the anti-CD3 antibody-adherent plate in an amount of 2.65 mL. At the same time, a plate to which a cell suspension not containing a retinoic acid had been added was also prepared. Each plate was cultured at 37°C for 4 days in a $CO_2$ incubator.

The PBMC which had been stimulated with an anti-CD3 antibody alone for 4 days without adding retinoic acid, and the PBMC which had been stimulated with an anti-CD3 antibody alone for 4 days in the presence of retinoic acid were each suspended at $0.1 \times 10^6$ cells and 2.0 mL/well in a medium containing 1% autologous plasma, 720 IU/mL IL-2, 0.2% HSA, 2.5 $\mu$g/mL Fungizone and 10 nM retinoic acid. The resulting suspension was seeded again on a 12-well plate for cell culture, and cultured at 37°C for 3 days in a $CO_2$ incubator. As a control, the PBMC which had been stimulated with an anti-CD3 antibody alone for 4 days without adding retinoic acid was suspended in a medium not containing retinoic acid at the same cell concentration, and similarly cultured.

On the 7th day after initiation of stimulation of the PBMC, each PBMC was suspended in a medium containing retinoic acid at the final concentration of 10 nM at $0.1 \times 10^6$ cells/mL and 2.0 mL/well, seeded on a 12-well plate for cell culture, and further cultured until the 10th day. The control was subjected to the same operation using a medium not containing retinoic acid, and similarly cultured until the 10th day.

On the 10th day, in each culture, cell surface markers in CD4 single positive cells and the proportion of IL-4 and IFN-$\gamma$-producing cells in CD4 single positive cells in response to PMA+ ionomycin stimulation were measured.

The proportion of IL-4 and IFN-$\gamma$-producing cells in the cultured cells in response to PMA+ ionomycin stimulation was measured. PMA at the final concentration of 10 ng/mL and ionomycin at the final concentration of 1 $\mu$g/mL were added to the cultured cells on the 10th day, and the cells were cultured for 1 hour. After Brefeldin A (manufactured by Sigma Aldrich) was added, the cells were further cultured for 3 hours, and the reaction was stopped with a 10-fold amount of cold PBS. These cells was stained with a FITC-labeled anti-human CD4 antibody (manufactured by Becton Dickinson) at room temperature for 15 minutes. Then, after cells were fixed and permeated using IntraPrep (manufactured by Beckman Coulter), the cells were stained with a PE-Cy7-labeled anti-human IFN-$\gamma$ antibody (manufactured by Becton Dickinson) and a PE-labeled anti-human IL-4 antibody (manufactured by Beckman Coulter) at room temperature for 20 minutes. After the cells were washed, flow cytometry analysis was carried out using

FACScant (manufactured by Becton Dickinson).

In each graph of Figs. 26 to 29, "RR" indicates the group of cells cultured with addition of retinoic acid from initiation of stimulation of PBMC to the 10th day, "OR" indicates the group of cells cultured without addition of retinoic acid from initiation of stimulation of PBMC to the 4th day and then cultured with addition of retinoic acid, and "O-" indicates the group of cells cultured without adding retinoic acid from initiation of stimulation of PBMC to the 10th day (control).

The results of measurement of cell surface markers in CD4 single positive cells are shown in Fig. 26. The proportion of memory T cells was measured by staining the cells with an anti-CD4 antibody, an anti-CD45RA antibody and an anti-CD62L antibody. In each cell group of Fig. 26, bars represent the proportions (%) of CD45RA+/CD62L+ (naive T-like cell), CD45RA-/CD62L+ (central memory T-like cell), CD45RA-/CD62L- (effector memory T-like cell) and CD45RA+/CD62L- (effector T-like cell) from left. As seen from Fig. 26, the proportions of central memory T-like cells and effector memory T-like cells were increased in the case of addition of retinoic acid, as compared with the case of no addition (O-) .

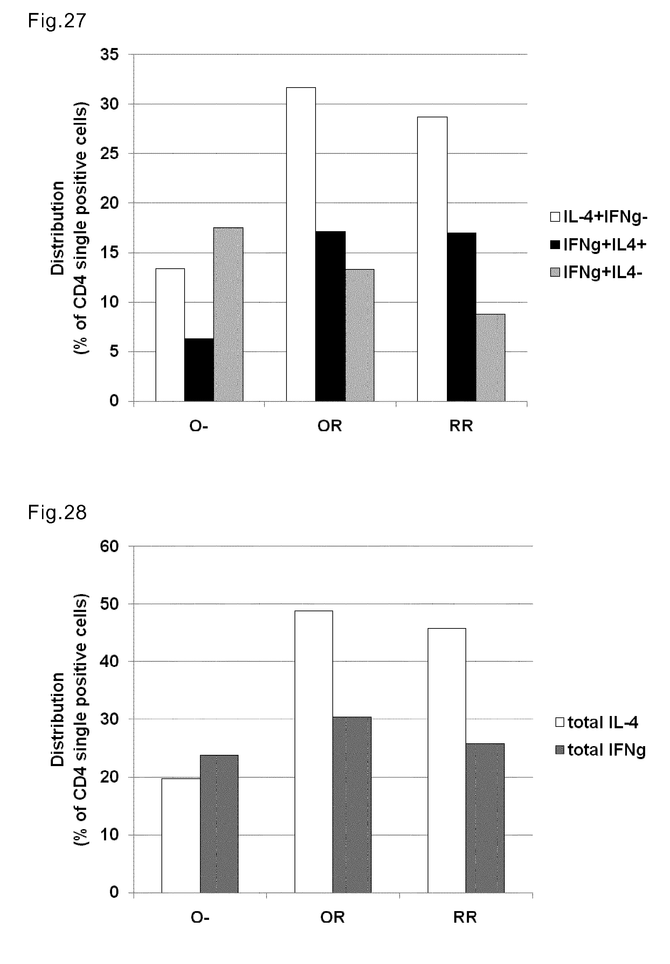

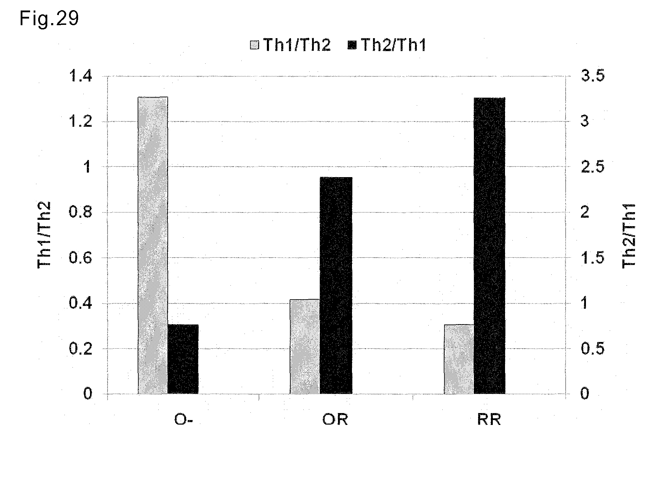

The results of the proportion of IL-4-producing or IL-4-nonproducing, and IFN-γ-producing or IFN-γ-nonproducing cells in CD4 single positive cells are shown in Fig. 27, and the results of the proportion of IL-4-producing and IFN-γ-producing cells in CD4 single positive cells are shown in Fig. 28. In addition, the ratio of the proportion of IL-4-producing and IFN-γ-nonproducing cells (Th2) and the proportion of IL-4-nonproducing and IFN-γ-producing cells (Th1) is shown in Fig. 29. In each cell group in Fig. 27, bars represent the proportions (%) of IL-4-producing and IFN-γ-nonproducing cells, IL-4-producing and IFN-γ-producing cells, and IL-4-nonproducing and IFN-γ-producing cells from left. In each cell group in Fig. 28, bars represent the proportions (%) of IL-4-producing cells, and IFN-γ-producing cells from left. In each cell group in Fig. 29, bars represent Th1/Th2 and Th2/Th1 from left, and in the scale of the ordinate axis, the left side (0 to 1.4) corresponds to Th1/Th2, and the right side (0 to 3.5) corresponds to Th2/Th1.

As a result, as seen from Fig. 28, the proportion of cells producing IL-4 which is a type II cytokine was high in "RR" and "OR", and the proportion of cells producing IFN-γ which is a type I cytokine was the highest in "OR". In addition, the proportion of IL-4-producing and IFN-γ-producing cells was high in "RR" and "OR", as compared with "O-" representing no addition. As seen from Fig. 29, since the Th2/Th1 ratio was higher in "RR" than in "OR", it was made clear that the presence of retinoic acid during culturing from initiation of stimulation of the PBMC to the 4th day was important for inducing a Th2-type helper T cell. From the above-mentioned results, it was found that CD4 single positive cells producing a type II cytokine or producing a type I cytokine could be induced by controlling the timing of addition of retinoic acid, like the CD8 single positive cells.

Industrial Applicability

According to the present invention, there can be provided a process for preparing a cell population containing a memory T-like cell, which is suitable for use in an immunotherapy, and a process for preparing a cell population in which the proportion of a memory T-like cell is increased and a desired gene is transferred with a high efficiency and is highly expressed. The cell population obtained by the process is extremely useful in treatment of diseases by cell therapy.

Sequence Listing Free Text

SEQ ID NO: 1; Fibronectin fragment named CH-271.
SEQ ID NO: 2; Fibronectin fragment named CH-296.
SEQ ID NO: 3; Fibronectin fragment named H-271.
SEQ ID NO: 4; Fibronectin fragment named H-296.

**Claims**

1.  A process for preparing a cell population containing a memory T-like cell, comprising a step of ex vivo culturing a cell population containing a T cell or a precursor cell of a T cell using a retinoic acid and a CD3 ligand.

2.  The process according to claim 1, wherein the step of ex vivo culturing a cell population containing a T cell or a precursor cell of a T cell is any step selected from (1) to (3):

    (1) a step of culturing a cell population containing a T cell or a precursor cell of a T cell in the presence of a retinoic acid and a CD3 ligand,
    (2) a step of culturing a cell population containing a T cell or a precursor cell of a T cell in the presence of a retinoic acid and a CD3 ligand and, then, culturing the cell population in the absence of a retinoic acid and a CD3 ligand, and
    (3) a step of culturing a cell population containing a T cell or a precursor cell of a T cell in the presence of a CD3 ligand and in the absence of a retinoic acid and, then, culturing the cell population in the presence of a retinoic acid.

3.  The process according to claim 1 or 2, wherein the step of ex vivo culturing a cell population containing a T cell or a precursor cell of a T cell is carried out in the presence of fibronectin or a fragment thereof or a mixture thereof.

4.  The process according to any one of claims 1 to 3, wherein the cell population containing a T cell or a precursor cell of a T cell is a peripheral blood mononuclear cell.

5.  The process according to any one of claims 1 to 4, further comprising a step of selectively recovering a memory T-like cell from the resulting cell population.

6. A cell population prepared by the process according to any one of claims 1 to 5.

7. A process for preparing a cell population into which a desired gene is transferred, comprising the steps of:

(a) a step of culturing a cell population containing a T cell or a precursor cell of a T cell by the process according to claim 1, and
(b) a step of transferring the desired gene into the cell population during the step (a) or after completion of the step (a).

8. The process according to claim 7, which is carried out by any one of the following steps (1) to (3):

(1) a step of culturing a cell population containing a T cell or a precursor cell of a T cell in the presence of a retinoic acid and a CD3 ligand, and transferring a desired gene into the resulting cell,
(2) a step of culturing a cell population containing a T cell or a precursor cell of a T cell in the presence of a retinoic acid and a CD3 ligand, transferring a desired gene into the resulting cell population, and then culturing the cell population in the absence of a retinoic acid and a CD3 ligand, and
(3) a step of culturing a cell population containing a T cell or a precursor cell of a T cell in the presence of a CD3 ligand and in the absence of a retinoic acid, transferring a desired gene into the resulting cell population, and then culturing the cell population in the presence of a retinoic acid.

9. The process according to claim 7 or 8, wherein the step of ex vivo culturing a cell population containing a T cell or a precursor cell of a T cell is carried out in the presence of fibronectin or a fragment thereof or a mixture thereof.

10. The process according to any one of claims 7 to 9, wherein the desired gene is transferred by a retrovirus vector.

11. The process according to claim 10, wherein the desired gene is transferred in the presence of fibronectin or a fragment thereof or a mixture thereof.

12. The process according to any one of claims 7 to 11, wherein the desired gene is a gene encoding a receptor recognizing an antigen.

13. The process according to claim 12, wherein the receptor recognizing an antigen is a T cell receptor.

14. The process according to any one of claims 7 to 13, wherein the cell population containing a T cell or a precursor cell of a T cell is a peripheral blood mononuclear cell.

15. A T cell population into which a desired gene is transferred, obtained by the process according to any one of claims 7 to 14.

Fig.1

Fig.2

Fig.3

Fig.4

Fig.5

Fig.6

Fig.7

Fig.8

Fig.9

Fig.10

Fig.11

Fig.12

Fig.13

Fig.14

Fig.15

Fig.16

Fig.17

Fig.18

Fig.19

Fig.20

Fig.21

Fig.22

Fig.23

Fig.24

Fig.25

Fig.26

Fig.27

Fig.28

Fig.29

## INTERNATIONAL SEARCH REPORT

| International application No. |
| --- |
| PCT/JP2010/064254 |

A. CLASSIFICATION OF SUBJECT MATTER
*C12N5/00*(2006.01)i, *C12N5/07*(2010.01)i, *C12N5/10*(2006.01)i, *C07K14/78*
(2006.01)n

According to International Patent Classification (IPC) or to both national classification and IPC

B. FIELDS SEARCHED

Minimum documentation searched (classification system followed by classification symbols)
C12N1/00-7/08, C07K14/78

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII), BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
| --- | --- | --- |
| X/Y | ALLENDE, L.M., et al., "Retinol(vitamin A) is a cofactor in CD3-induced human T-lymphocyte activation" Immunology, 1997, Vol.90, p.388-396 | 1,2,4-6/3, 7-15 |
| Y | Kazuki OSUMI, Teruaki SEKINE, "Kasseika Zofuku Baiyo ni yoru Lymph-kyu no Zoshoku Hannosei to T-Saibo Subset no Henka", Dai 22 Kai Japan Society for Biological Therapy Gakujutsu Shukai Sokai, 01 November 2009 (01.11.2009), page 108 | 1-15 |
| Y | CHRAPUSTA, S.J., st al., "Immune abnormalities in Aneurysmal Subarachnoid Haemorrhage patients: Relation to delayed cerebral vasospan" Scand.J. Immunol., 2000, Vol.51, p.400-407 | 1-15 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 28 October, 2010 (28.10.10) | 09 November, 2010 (09.11.10) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/064254

C (Continuation). DOCUMENTS CONSIDERED TO BE RELEVANT

| Category* | Citation of document, with indication, where appropriate, of the relevant passages | Relevant to claim No. |
|---|---|---|
| A | KIM Y-M., et al., "Ex vivo expansion of human umbilical cord blood derived T-lymphocytes with homologous cord blood plasma" Tohoku.J.Exp.Med., 2005, Vol.205, p.115-122 | 1-15 |
| A | YAMAGUCHI, B., et al., "CD4$^+$-central memory and effector memory T cells in patients with Asthma" Dokkyo J. Med.Sci., 2006, Vol.33, No.1, p.1-10 | 1-15 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009194444 A
- JP 2009287258 A
- WO 03016511 A
- WO 03080817 A
- WO 2005019450 A
- WO 00018 A
- JP 2005336062 A

- WO 9718318 A
- WO 0009168 A
- WO 9207943 A
- WO 9526200 A
- WO 200811150 A
- WO 2008111506 A

**Non-patent literature cited in the description**

- *Nat. Immunol.,* 2000, vol. 1, 47-53
- *Nature,* 1999, vol. 401, 708-712
- *J. Clin. Invest.,* 2005, vol. 115 (6), 1616-1626
- *J. Immunol.,* 2005, vol. 175 (2), 739-748
- **Ruoslahti E. et al.** *J. Biol. Chem.,* 1981, vol. 256 (14), 7277-7281
- *J. Biochem.,* 1991, vol. 110, 284-291
- *Science,* 1979, vol. 206, 347-349
- **Fowler D. H.** *Leukemia and Lymphoma,* 2000, vol. 38, 221-234

- **Morgenstern J. P. ; Land H.** *Nucleic Acids Research,* 1990, vol. 18 (12), 3587-3596
- *Proceedings of the National Academy of Sciences of the USA,* 1988, vol. 85, 6460-6464
- Molecular Cloning: A Laboratory Manual. Cold Spring Harbor Laboratory, 2001
- *Clinical Cancer Research,* 2005, vol. 11, 5581-5589